(19) Europäisches Patentamt
European Patent Office
Office européen des brevets

(11) EP 0 740 252 A2

(12) EUROPEAN PATENT APPLICATION

(43) Date of publication:
30.10.1996 Bulletin 1996/44

(51) Int Cl.[6]: G06F 13/00

(21) Application number: 96302467.4

(22) Date of filing: 09.04.1996

(84) Designated Contracting States:
AT DE FR GB

(30) Priority: 07.04.1995 JP 108306/95
07.09.1995 JP 230365/95
07.09.1995 JP 230387/95
08.11.1995 JP 289961/95

(71) Applicant: KUREHA KAGAKU KOGYO KABUSHIKI KAISHA
Tokyo 103 (JP)

(72) Inventors:
- Kikuchi, Tatsuji
  Tokyo (JP)
- Nitta, Toyohiko
  Kitasoma-gun, Ibaraki (JP)
- Ito, Tomoko
  Tokyo (JP)
- Kasai, Kenji
  Atsugi-shi, Kanagawa (JP)
- Chiba, Yukinobu
  Tokyo (JP)

(74) Representative:
Cross, Rupert Edward Blount et al
BOULT, WADE & TENNANT
27 Furnival Street
London EC4A 1PQ (GB)

# (54) Apparatus, method, and standard curve for measuring clearance

(57) A clearance measuring apparatus for measuring a clearance (CL) on the basis of a dose (D) of the test substance, a blood collection time (T) required from administration of the test substance to blood collection, and a test substance concentration (Cp) in plasma obtained from blood collected, said apparatus comprising:

storage means for storing a first standard function expressed by:

$$Cp/D = A \times \exp(-\alpha \times (CL \times T)) + B \times \exp(-\beta \times (CL \times T))$$

input means for receiving inputs of (D), (T) and (Cp); and

clearance calculation means for calculating (CL) by applying (D), (T) and (Cp) to the first standard function.

Fig.1

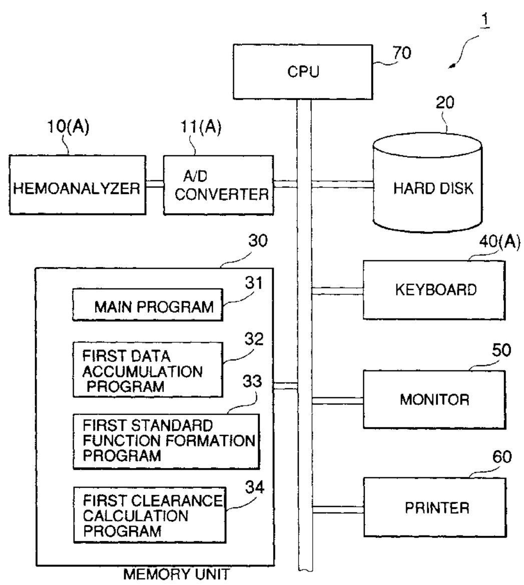


EP 0 740 252 A2

## Description

### BACKGROUND OF THE INVENTION

#### Field of the Invention

The present invention relates to a clearance measurement apparatus and method for measuring a clearance expressed as a flow rate when the excretion amount of a test substance in urine per unit time is converted into a concentration in plasma.

Further, the present invention relates to a clearance measurement storage medium (computer program product) which stores a clearance measurement program for measuring a clearance expressed as a flow rate when the excretion amount of a test substance in urine per unit time is converted into a concentration in plasma.

Furthermore, the present invention relates to a clearance measurement standard curve. The standard curve according to the present invention is formed by plotting specific indices along the ordinate and abscissa. A renal clearance can be accurately measured by single administration of a test substance and single blood collection of a sample.

#### Related Background Art

A glomerular filtration rate (GFR) is used as one index of the renal function. As one of the GFR Golden Standards, a measurement method using an inulin clearance is known. Inulin is a straight-chain fructan consisting of β-D-(2-1) fructose and having nonreducible glucose at its terminal. Inulin is stored in the rootstocks of a dahlia and a Jerusalem artichoke. Inulin is a white amorphous powder having a molecular weight of about 5,000. Inulin is an ideal GFR measurement substance because it is not bound with proteins in plasma, or it is not excreted or absorbed again in uriniferous tubules except for glomeruli.

The following three conventional methods are proposed for measuring the inulin clearance. The first method is described in "Renal Function Test of Modern Clinical Function Tests--glomerular filtration rate (Yasuyoshi Mito et al., Nihon Rinsho, Vol. 37, Special Summer Edition, pp. 1,283 - 1,284, 1979)". According to this reference, a 3% inulin solution is prepared the day before the test, and 50 mg/kg of inulin is subjected to intravenous injection over 30 min so as to distribute the inulin in a concentration of 25 mg/dℓ for an extracellular humor amount (20% the body weight) for the first administration. Inulin in a maintenance amount upon introducing a urethral catheter (GFR × 0.25 mg/min wherein GFR is predicted from the intrinsic creatinine clearance) is continuously subjected to intravenous injection for 90 min. Blood collection and urine collection are repeated every 30 min, and the inulin is quantitatively measured in accordance with the anthrone method. The clearance is calculated in accordance with the inulin concentration ratio.

According to the second method, after inulin is subjected to single intravenous injection, blood is collected seven or more times over time without any urine collection. The inulin concentration in plasma is measured, and the inulin clearance is calculated in accordance with a two-compartment model (e.g., see K.W. Florijn et al., Kidney International, Vol. 46, pp. 252 - 259, 1994).

In addition, according to the third method, after inulin (5 g) is subjected to single intravenous injection, the inulin concentration in plasma, obtained in single blood collection, is measured. The inulin clearance is calculated in accordance with a one-compartment model (K. Jung et al., Nephron, Vol. 59, pp. 694 - 695, 1991).

According to the first method described above, however, continuous intravenous injection of inulin is required, and blood must be frequently collected. In addition, strictly timed urine collection and perfect excretion of urine are required. For these reasons, the load on a test subject is disadvantageously heavy. In particular, urine is generally and clinically collected by natural excretion. Errors tend to occur in test subjects who make it difficult to accurately collect urine (an uncooperative patient, a patient who has difficulty in urinating, a patient who forgets urine collection due to an urgent business or the like, an infant, and small animals such as a mouse and a guinea pig), thus posing a problem.

According to the second method described above, however, blood must be frequently collected at predetermined times. As in the first method, the load on the test subject is disadvantageously heavy.

According to the third method, measurement can be performed by single intravenous injection and single blood collection to reduce the load on the test subject. The third method, however, has disadvantageously large errors. More specifically, when the concentration of inulin in plasma after intravenous injection is plotted as a function of time, a single line expected from the one-compartment model cannot be obtained, but two lines of $\alpha$ and $\beta$ phases are obtained by approximation. In this manner, the approximation using the one-compartment model is poor in accuracy.

A clearance is a flow rate obtained when the excretion amount of a test substance in urea per unit time is converted into a test substance concentration in plasma.

In the standard method, an inulin clearance (CL) is the product of a urine flow rate (V) and a ratio of an inulin concentration (U) in urine to an inulin concentration (Cp) in plasma and can be obtained by the following equation:

$$CL = (U/Cp) \times V.$$

In this case, in addition to the pains resulting from continuous inulin intravenous injection and frequent blood collection, strict timed urine collection and perfect excretion of urine are required. Since the test is ideally conducted under insertion of a bladder catheter, a heavy load is imposed on the patient. This measurement method is a cumbersome measuring method even to the physician. In addition, urine is generally and clinically collected by natural excretion. Errors tend to occur in test subjects who make it difficult to accurately collect urine (an uncooperative patient, a patient who has difficulty in urinating, a patient who forgets urine collection due to an urgent business or the like, and an infant).

A method by means of single intravenous injection and frequent blood collection using a two-compartment model measures the clearance from only the concentration in plasma without requiring urine collection. If inulin is perfectly excreted by only glomerular filtration, the total amount of the test substance excreted in urine must be equal to the dose (D) of the test substance. When the logarithm of the concentration (Cp) of the test substance in plasma is plotted as a function of time, the concentration in plasma can be approximated to the two-compartment model consisting of the $\alpha$ phase as the distribution phase and the $\beta$ phase as the excretion phase. The inulin clearance (CL) can be obtained by the following equation using a ratio of the dose (D) of the inulin and the area (AUC) under the curve of the concentration in plasma:

$$CL = D/AUC.$$

In this case, urine collection is not imposed on the patient, but 20 m$\ell$ (inulin: about 5 g) of an injection solution, for example, is administered over about 5 min, and blood is frequently collected at predetermined collection timings, thus imposing the heavy load on the patient.

According to the method of K. Jung et al. by means of single intravenous injection and single blood collection using a one-compartment model, the isotope clearances of 21 patients suffering from chronic renal diseases are measured using $^{99m}$Tc-DTPA (diethylenetriaminepentaacetate), and factors $k_t$ for the respective times are obtained from the isotope clearances, the inulin dose, and the inulin concentrations of the respective times. The products of the factors $k_t$ and the inulin dose are divided by the inulin concentrations to obtain inulin clearances for the respective times. The regression analysis is performed between the inulin clearances and the DTPA values to determine an optimal factor $k_0$. The product of the optimal factor $k_0$ and the inulin dose is divided by the inulin concentration of one sample to obtain the inulin clearance.

In this case, this requires time-consuming measurement of the isotope clearances and inulin clearances of a given number of patients in advance. The inulin concentrations in plasma upon intravenous injection are plotted as a function of time, one straight line expected from the one-compartment model cannot be approximated, but two straight lines of $\alpha$ and $\beta$ phases are approximated. The approximation using the one-compartment model has apparently a limitation on accuracy.

## SUMMARY OF THE INVENTION

It is an object of the present invention to provide an apparatus, a method, a computer program product and a standard curve for measuring clearance, which are capable of solving the conventional problems described above, reducing the load on a test subject, and allowing highly accurate measurement.

The present inventors have made extensive studies to develop a new clearance measuring method free from the above conventional problems and have obtained the following findings. Clearances (CL) are calculated from the two-compartment model using inulin concentrations (Cp) in plasma of blood collected at the respective times (T) upon single inulin intravenous injection (dose D) to a large number of rats, dogs, and humans having renal diseases of various levels as well as healthy rats, dogs and humans. Using these clearance (CL) values, the product of CL and T is plotted along one of the coordinate axes, and a ratio value of Cp to D is plotted along the other of the coordinate axes. Surprisingly, the plotted points converge to one curve ($CL \times T$ - Cp/D curve) for each animal species. Using this $CL \times T$ - Cp/D curve, a clearance can be highly accurately obtained from the inulin concentration in plasma of each sample collected at arbitrary time upon single inulin intravenous injection.

The present inventors also have the following findings. Using a steady-state distribution volume (or total distribution volume) (Vss) obtained from the two-compartment model, $CL \times T/Vss$ is plotted along one of the coordinate axes, while Cp/(D/Vss) is plotted along the other of the coordinate axes. In this case, the plotted points converge to one curve [$CL \times T/Vss$ - Cp/(D/Vss) curve] for each animal species. Using this $CL \times T/Vss$ - Cp/(D/Vss) curve, a clearance can be highly accurately obtained from the inulin concentration in plasma of each sample collected at arbitrary time upon single inulin intravenous injection.

The present inventors have also found that the same result can be obtained even in use of meglumine iothalamate in place of inulin.

The present invention are based on the above findings.

The first clearance measuring apparatus of the present invention is capable of measuring a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said apparatus comprising:

storage means for storing a first standard function which takes, as one parameter, a product value of the clearance (CL) and the blood collection time (T) and as the other parameter, a value obtained by dividing the test substance concentration (Cp) by the dose (D);

input means for receiving inputs of the dose (D), the blood collection time (T), and the test substance concentration (Cp); and

clearance calculation means for calculating the clearance (CL) by applying the dose (D), the blood collection time (T) and the test substance concentration (Cp) received by said input means to the first standard function stored in said storage means.

According to the first clearance measuring apparatus of the present invention, the clearance calculation means is used to easily and accurately calculate the clearance (CL) by applying the dose (D), the blood collection time (T), and the test substance concentration (Cp) to the first standard function. The dose (D), the blood collection time (T), and the test substance concentration (Cp) applied to the first standard function are data obtained by performing single administration of the test substance and single blood collection. More specifically, the dose (D) is an amount at which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. Since all the data necessary for the first clearance measuring apparatus of the present invention can be obtained by single administration of the test substance and single blood collection, the load of blood collection and the like on the test subject is small.

In this case, the first standard function is preferably expressed by, e.g., the following equation:

$$Cp/D = A \times \exp(-\alpha \times (CL \times T)) + B \times \exp(-\beta \times (CL \times T))$$

(where A, B, $\alpha$ and $\beta$ are constants specified for each animal species).

Further, the second clearance measuring apparatus of the present invention is capable of measuring a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said apparatus comprising:

storage means for storing a second standard function which takes, as one parameter, a value obtained by dividing a product value of the clearance (CL) and the blood collection time (T) by the steady-state distribution volume (Vss) and, as the other parameter, a value obtained by dividing the test substance concentration (Cp) by a value obtained by dividing the dose (D) by the steady-state distribution volume (Vss);

input means for receiving inputs of the dose (D), the blood collection time (T), the test substance concentration (Cp) and the steady-state distribution volume (Vss); and

clearance calculation means for calculating the clearance (CL) by applying the dose (D), the blood collection time (T), the test substance concentration (Cp) and the steady-state distribution volume (Vss) received by said input means to the second standard function stored in said storage means.

According to the second clearance measuring apparatus of the present invention, the clearance calculation means is used to easily and accurately calculate the clearance (CL) by applying the dose (D), the blood collection time (T), the test substance concentration (Cp), and the steady-state distribution volume (Vss) to the second standard function. The dose (D), the blood collection time (T), the test substance concentration (Cp), and the steady-state distribution volume (Vss) applied to the second standard function are data obtained by performing measurement of the body weight of the test subject, single administration of the test substance, and single blood collection. More specifically, the dose (D) is an amount at which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. In addition, the steady-state distribution volume (Vss) is a volume of the humor in which the administrated test substance is diffused and distributed in the living body and can be generally obtained by multiplying the weight of the test subject by a predetermined ratio (e.g., 20%). Since all the data necessary for the second clearance measuring apparatus of the present invention can be obtained by single administration of the test substance and single blood collection, the load of blood collection and the like on the test subject is small.

In this case, the second standard function is preferably expressed by, e.g., the following equation:

$$Cp/(D/Vss) = A \times \exp(-\alpha \times (CL \times T)/Vss) + B \times \exp(-\beta \times (CL \times T)/Vss)$$

(where A, B, $\alpha$ and $\beta$ are constants specified for each animal species).

The first clearance measuring method of the present invention enables to measure a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said method comprising the step of:

calculating the clearance (CL) by applying the dose (D), the blood collection time (T) and the test substance concentration (Cp) to a first standard function which takes, as one parameter, a product value of the clearance (CL) and the blood collection time (T) and, as the other parameter, a value obtained by dividing the test substance concentration (Cp) by the dose (D).

According to the first clearance measuring method of the present invention, the clearance (CL) can be easily and accurately calculated by applying the dose (D), the blood collection time (T), and the test substance concentration (Cp) to the first standard function. The dose (D), the blood collection time (T), and the test substance concentration (Cp) applied to the first standard function are data obtained by performing single administration of the test substance and single blood collection. More specifically, the dose (D) is an amount at which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. Since all the data necessary for the first clearance measuring method of the present invention can be obtained by single administration of the test substance and single blood collection, the load of blood collection and the like on the test subject is small.

In this case, the first standard function is preferably expressed by, e.g., the following equation:

$$Cp/D = A \times \exp(-\alpha \times (CL \times T)) + B \times \exp(-\beta \times (CL \times T))$$

(where A, B, $\alpha$ and $\beta$ are constants specified for each animal species).

Further, the second clearance measuring method of the present invention enables to measure a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said method comprising the step of:

calculating the clearance (CL) by applying the dose (D), the blood collection time (T), the test substance concentration (Cp) and the steady-state distribution volume (Vss) to a second standard function which takes, as one parameter, a value obtained by dividing a product value of the clearance (CL) and the blood collection time (T) by the steady-state distribution volume (Vss) and, as the other parameter, a value obtained by dividing the test substance concentration (Cp) by a value obtained by dividing the dose (D) by the steady-state distribution volume (Vss).

According to the second clearance measuring method of the present invention, the clearance (CL) can be easily and accurately calculated by applying the dose (D), the blood collection time (T), the test substance concentration (Cp), and the steady-state distribution volume (Vss) to the second standard function. The dose (D), the blood collection time (T), the test substance concentration (Cp), and the steady-state distribution volume (Vss) applied to the second standard function are data obtained by performing measurement of the body weight of the test subject, single administration of the test substance, and single blood collection. More specifically, the dose (D) is an amount at which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. In addition, the steady-state distribution volume (Vss) is a volume of the humor in which the administrated test substance is diffused and distributed in the living body and can be generally obtained by multiplying the weight of the test subject by a predetermined ratio (e.g., 20%). Since all the data necessary for the second clearance measuring method of the present invention can be obtained by single administration of the test substance and single blood collection, the load of blood collection and the like on the test subject is small.

In this case, the second standard function is preferably expressed by, e.g., the following equation:

$$Cp/(D/Vss) = A \times \exp(-\alpha \times (CL \times T)/Vss) + B \times \exp(-\beta \times (CL \times T)/Vss)$$

(where A, B, $\alpha$ and $\beta$ are constants specified for each animal species).

The first computer program product (the first clearance measurement storage medium) of the present invention is used with an information processing apparatus, said computer program product comprising:

a computer usable medium having a program area and a computer readable program embodied in said program area of said medium for measuring a clearance, said computer program product having:

a computer readable input routine for causing said computer to receive inputs of a dose (D) of a test substance administered to a test subject, a blood collection time (T) required from administration of the test substance to blood collection with respect to the test subject, and a test substance concentration (Cp) in plasma which is obtained from blood collected from the test subject, and

a computer readable arithmetic routine for causing said computer to apply the dose (D), the blood collection time (T) and the test substance concentration (Cp) input in the input routine to a first standard function which takes, as one parameter, a product value of a clearance (CL) of the test substance and the blood collection time (T) and, as the other parameter, a value obtained by dividing the test substance concentration (Cp) by the dose (D), thereby calculating the clearance (CL).

The first computer program product (the first clearance measurement storage medium) of the present invention is loaded in a predetermined information processing apparatus, and a clearance measurement program stored in a program area is read to allow the information processing apparatus to execute the clearance measurement program. Upon this execution, a dose (D), a blood collection time (T), and a test substance concentration (Cp) are applied to a first standard function, so that a clearance (CL) can be easily and highly accurately calculated. The dose (D), the blood collection time (T), and the test subject concentration (Cp) applied to the first standard function are data obtained by single administration of the test substance and single blood collection with respect to the test subject. More specifically, the dose (D) is an amount in which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. Since all the data necessary for calculating the clearance (CL) can be obtained by single administration of the test substance and single blood collection, the load of blood collection and the like on the test subject is small.

In this case, the first standard function is preferably expressed by, e.g., the following equation:

$$Cp/D = A \times \exp(-\alpha \times (CL \times T)) + B \times \exp(-\beta \times (CL \times T))$$

(where A, B, $\alpha$ and $\beta$ are constants specified for each animal species).

Further, the second computer program product (the second clearance measurement storage medium) of the present invention is used with an information processing apparatus, said computer program product comprising:

a computer usable medium having a program area and a computer readable program embodied in said program area of said medium for measuring a clearance, said computer program product having:

a computer readable input routine for causing said computer to receive inputs of a dose (D) of a test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, and

a computer readable arithmetic routine for causing said computer to apply the dose (D), the blood collection time (T), the test substance concentration (Cp) and the steady-state distribution volume (Vss) input in the input routine to a second standard function which takes, as one parameter, a value obtained by dividing a product value of a clearance (CL) of the test substance and the blood collection time (T) by the steady-state distribution volume (Vss) and, as the other parameter, a value obtained by dividing the test substance concentration (Cp) by a value obtained by dividing the dose (D) by the steady-state distribution volume (Vss), thereby calculating the clearance (CL).

The second computer program product (the second clearance measurement storage medium) of the present invention is loaded in a predetermined information processing apparatus, and a clearance measurement program stored in a program area is read to allow the information processing apparatus to execute the clearance measurement program. Upon this execution, a dose (D), a blood collection time (T), a test substance concentration (Cp), and a steady-state distribution volume (Vss) are applied to a second standard function, so that a clearance (CL) can be easily and highly accurately calculated. The dose (D), the blood collection time (T), the test subject concentration (Cp), and the steady-state distribution volume (Vss) applied to the second standard function are data obtained by measurement of a body weight, single administration of the test substance, and single blood collection to the test subject. More specifically, the dose (D) is an amount in which the test substance is administered to the test subject. The blood collection time (T) is a time required from administration of the test substance to blood collection with respect to the test subject. The test substance concentration (Cp) is the concentration of the test substance in plasma, which is obtained from blood collected from the test subject. In addition, the steady-state distribution volume (Vss) is a volume of the humor in which the administrated test substance is diffused and distributed in the living body and can be generally obtained by multiplying the weight of the test subject by a predetermined ratio (e.g., 20%). Since all the data necessary for calculating the clearance (CL) can be obtained by measurement of the body weight, single administration of the test substance, and single blood collection, the load of blood collection and the like on the test subject is small.

In this case, the second standard function is preferably expressed by, e.g., the following equation:

$$Cp/(D/Vss) = A \times \exp(-\alpha \times (CL \times T)/Vss)$$
$$+ B \times \exp(-\beta \times (CL \times T)/Vss)$$

(where A, B, $\alpha$ and $\beta$ are constants specified for each animal species).

Each of the first and second standard functions may be any function using, e.g., a product value (CL × T) of the clearance (CL) and the blood collection time (T) and a quotient value (Cp/D) obtained by dividing the test substance concentration (Cp) by the dose (D) as two parameters to substantially uniquely determine the relationship between the values of these parameters. For this reason, the first and second standard functions may be mathematical expressions or numerical tables. In addition, the first and second standard functions may be curves having these two parameters as coordinate axes.

The first clearance measurement standard curve of the present invention is used for measuring a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said curve characterized in that a product value of the time (T) required from administration of a test substance to blood collection and the clearance (CL) is plotted along one of coordinate axes, and a ratio value of the test substance concentration (Cp) in plasma to the test substance dose (D) is plotted along the other of the coordinate axes.

Further, the second clearance measurement standard curve of the present invention is used for measuring a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said curve characterized in that a value obtained by dividing a product value of the time (T) required from administration of a test substance to blood collection and the clearance (CL) by the steady-state distribution volume (Vss) is plotted along one of coordinate axes, and a value obtained by dividing the test substance concentration (Cp) in plasma by a ratio value of the test substance dose (D) to the steady-state distribution volume (Vss).

The present invention will become more fully understood from the detailed description given hereinbelow and the accompanying drawings which are given by way of illustration only, and thus are not to be considered as limiting the present invention.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

## BRIEF DESCRIPTION OF THE DRAWINGS

Fig. 1 is a block diagram showing the arrangement of a clearance measuring apparatus according to the first embodiment of the present invention.

Fig. 2 is a flow chart showing a method for measuring by the use of a clearance measuring apparatus according to the first embodiment of the present invention.

Fig. 3 is a flow chart showing a method for calculating a clearance.

Fig. 4 is a block diagram showing the data structure of a clearance measurement storage medium according to the first embodiment of the present invention.

Fig. 5 is a block diagram showing the arrangement of an information processing apparatus applied to the first and second embodiments of the present invention.

Fig. 6 is a perspective view showing an information processing apparatus applied to the first and second embodiments of the present invention.

Fig. 7 is a flow chart showing a processing flow of each program stored in the clearance measurement storage medium according to the first embodiment of the present invention.

Fig. 8 is a block diagram showing the arrangement of a clearance measuring apparatus according to the second embodiment of the present invention.

Fig. 9 is a flow chart showing a method for measuring by the use of a clearance measuring apparatus according to the second embodiment of the present invention.

Fig. 10 is a block diagram showing the data structure of a clearance measurement storage medium according to the second embodiment of the present invention.

Fig. 11 is a flow chart showing a processing flow of each program stored in the clearance measurement storage medium according to the second embodiment of the present invention.

Fig. 12 is a graph showing a first standard function [CL x T - Cp/D curve] for rats.

Fig. 13 is a graph showing a first standard function [CL × T - Cp/D curve] for dogs.

Fig. 14 is a graph showing a first standard function [CL × T - Cp/D curve] for humans.

Fig. 15 is a graph showing a second standard function [CL × T/Vss - Cp/(D/Vss) curve] for rats.

Fig. 16 is a graph showing a second standard function [CL × T/Vss - Cp/(D/Vss) curve] for dogs.

Fig. 17 is a graph showing a second standard function [CL × T/Vss - Cp/(D/Vss) curve] for humans.

Fig. 18 is a graph showing a first standard function [CL × T - Cp/D curve] for dogs in use of meglumine iothalamate as a test substance.

## DESCRIPTION OF THE PREFERRED EMBODIMENT

(First Embodiment)

A preferred embodiment of the present invention will be described with reference to the accompanying drawings. Note that the same reference numerals in the following description denote the same parts, and a repetitive description will be omitted. Fig. 1 is a block diagram showing the arrangement of a clearance measuring apparatus 1 according to the first embodiment of the present invention. The clearance measuring apparatus 1 is an apparatus for applying, to a first standard function $Cp/D = f_1(CL \times T)$, a dose (D) of a test substance administered once to a test subject, a blood collection time (T) required from administration of the test substance to the test subject to single blood collection, and a test substance concentration (Cp) in a plasma sample obtained upon preparing blood collected from the test subject, thereby obtaining a clearance (CL) serving as an excretion amount of the test substance in urine per unit time.

The clearance measuring apparatus 1 is an apparatus utilizing the characteristics of the first standard function $Cp/D = f_1(CL \times T)$ having excellent characteristics; it converges to an almost one type of standard function for each animal species, thereby highly accurately obtaining the clearance (CL) by single administration of the test substance and single blood collection. The characteristics of the first standard function are the facts found by the present inventor for the first time.

Examples of the test subject are mammals such as a mouse, a rat, a dog, a monkey, a guinea pig, and a human.

In addition, examples of the test substance are inulin and meglumine iothalamate. The first standard function is preferably exemplified as follows:

$$Cp/D = A \times \exp(-\alpha \times (CL \times T)) + B \times \exp(-\beta \times (CL \times T))$$

(where A, B, $\alpha$ and $\beta$ are constants specified for each animal species).

As shown in Fig. 1, the clearance measuring apparatus 1 comprises a hemoanalyzer 10 for analyzing the test substance concentration (Cp) of a plasma sample obtained upon preparing blood collected from the test subject, an A/D converter 11 for converting test substance concentration (Cp) as analog data output from the hemoanalyzer 10 into digital data, and a hard disk (storage means) 20 for storing data such as the test substance concentration (Cp) converted into the digital data and the first standard function data.

The clearance measuring apparatus 1 also comprises a memory unit 30 storing a plurality of processing programs, a keyboard 40 for inputting data such as the dose (D) of the test substance administered to the test subject, the blood collection time required from administration of the test substance to blood collection with respect to the test subject, and a test name (e.g., a test subject name and blood collection date), and a monitor 50 for displaying the clearance of the test subject together with input data. The clearance measuring apparatus 1 further comprises a printer 60 for printing out the clearance of the test subject and the like, and a CPU 70 for controlling the hemoanalyzer 10, the hard disk 20, the memory unit 30, and the like.

The memory unit 30 stores a first data accumulation program 32 for storing a plurality of measurement data consisting of the dose (D) of the test substance, the blood collection time (T), and the test substance concentration (Cp) in the hard disk 20, a first standard function formation program 33 for forming a first standard function on the basis of the plurality of measurement data stored in the hard disk 20, a first clearance calculation program (clearance calculation means) 34 for applying the dose (D), the blood collection time (T), and the test substance concentration (Cp) to the first standard function to calculate the clearance (CL), and a main program 31 for controlling the programs 32 to 34.

Note that the hemoanalyzer 10, the A/D converter 11, and the keyboard 40 constitute an input unit (input means) A for receiving the inputs of the dose (D), the blood collection time (T), and the test substance concentration (Cp). The test substance concentration (Cp) may be detected using an external hemoanalyzing unit, and this test substance concentration may be input from the keyboard 40. In this case, the hemoanalyzer 10 and the A/D converter 11 can be omitted. Only the keyboard 40 constitutes the input unit (input means) A. The dose (D), the blood collection time (T), and the test substance concentration (Cp) may be externally input through a modem and a communication line (neither is shown). In this case, the modem serves as the input unit (input means) A. Alternatively, the dose (D), the blood collection time (T), and the test substance concentration (Cp) may be input using a flexible disk unit (not shown). In this case, the flexible disk unit constitutes the input unit (input means) A.

Next, a clearance measuring method according to the first embodiment of the present invention using the clearance measuring apparatus 1 will be described with reference to a flow chart in Fig. 2. When an operator inputs an operation start instruction using the keyboard 40, the main program 31 is activated. In the main program 31, a selection menu for selecting execution of first standard function formation processing or execution of clearance calculation processing is displayed on the monitor 50 (step 102). When the operator inputs a processing selection instruction, it is determined whether this input represents the first standard function formation processing or the clearance calculation processing (step 103). If the input instruction is an instruction for selecting the first standard function formation processing, the main program 31 executes the first data accumulation program 32 (step 104).

Upon execution of the first data accumulation program 32, n sets of measurement data consisting of the dose (D) of the test substance, the blood collection times ($T_i$, i = 1 to n), and the test substance concentrations ($Cp_i$, i = 1 to n) are stored in the hard disk 20. More specifically, the test substance is administered to the test subject by single intravenous injection, and blood collection is normally performed at four or more time points during 4 to 8 hours after this intravenous injection. When the first data accumulation program 32 is executed, the hemoanalyzer 10 measures the test substance concentrations ($Cp_i$) of the plasma samples obtained upon preparing the collected blood.

Each test substance concentration ($Cp_i$) thus measured is converted into digital data by the A/D converter 11. When the blood collection time ($T_i$) and the dose (D) of the test substance which correspond to this test substance concentration ($Cp_i$) are input using the keyboard, these data are stored in the hard disk 20 as the measurement data. These measurement data are obtained for each blood collection operation, and a plurality of sets of measurement data are stored in the hard disk 20. In this case, a test substance concentration ($Cp_i$) obtained using an external hemoanalyzing unit may be input from the keyboard 40. When execution of the first data accumulation program 32 is completed, the main program 31 causes to execute the first standard function formation program 33 (step 105). Upon execution of the first standard function formation program 33, the clearance (CL) is obtained from the plurality of sets of measurement data stored in the hard disk 20, thereby forming a first standard function.

This clearance (CL) is formed in accordance with the following "clearance formation technique using a two-compartment model (to be referred to as an AUC method hereinafter)". According to the AUC method, the test substance concentrations ($Cp_i$) and the blood sampling times ($T_i$) of the measurement data are read out from the hard disk 20, and the test substance concentrations ($Cp_i$) are plotted as a function of the blood collection time ($T_i$). The clearance is obtained using the dose (D) of the test substance and the area (AUC) underneath the resultant approximate curve:

$$CL = D/AUC.$$

The formation of the approximate curve and the calculation of the AUC value are performed using a known technique described in, e.g., "Kiyoshi Yamaoka, Intracorporeal Dynamic Analysis Method of Drugs Using Microcomputer, pp. 100 - 105, Nanko-do (1984)".

It is preferable to perform calculation processing of the clearance (CL) using this AUC method for a maximum number of specimens having different blood collection times of each individual. In addition, it is also preferable to perform calculation processing of the clearance (CL) for a maximum number of individuals belonging to each animal species. The constants A, B, $\alpha$ and $\beta$ of the first standard function $Cp/D = A \times \exp(-\alpha \times (CL \times T)) + B \times \exp(-\beta \times (CL \times T))$ are calculated using the method of least squares so as to minimize the errors on the basis of the resultant clearances (CL) of each individual, and the blood collection times ($T_i$), the test substance concentrations ($Cp_i$), and the dose (D) which correspond to these clearances (CL), thereby obtaining the first standard function.

The two-compartment model described in Yamaoka's reference will be briefly described below. The administered test substance enters into a portion (compartment 1) regarded to be almost in equilibrium with the circulation system of the body and reaches an equilibrium. The test substance then shifts (velocity constant $k_{12}$) to a portion (compartment 2) which is not in equilibrium with the circulation system of the body, shifts (velocity constant $k_{21}$) from compartment 2 to compartment 1, and disappears (velocity constant $k_e$) from compartment 1. Therefore, the disappearance formula of the test substance from compartments 1 and 2 is solved to derive the following equation:

$$Cp = M \times \exp(-\mu T) + N \times \exp(-\nu T)$$

where M, N, $\mu$ and $\nu$ are constants. The distribution volume (V1) of compartment 1 is defined as $V1 = D/(M + N)$. Similarly, the distribution volume (V2) of compartment 2 is defined as $V2 = V1 \times k_{12}/k_{21}$. The steady-state distribution volume (Vss) can be calculated by $Vss = V1 + V2$. Furthermore, $AUC = M/\mu + N/\nu$.

When the input instruction is an instruction for selecting the clearance calculation processing in step 103 of the flow chart in Fig. 2, the main program 31 executes the first clearance calculation program 34 (step 106). A clearance of the test substance can be calculated by the first clearance calculation program 34. More specifically, the test substance is administered to the test subject once, and blood is collected once a predetermined period of time after the administration. Upon execution of the first clearance calculation program 34, the hemoanalyzer 10 detects the test substance concentration (Cp) in each plasma sample upon preparing the collected blood.

The test substance concentration (Cp) thus detected is converted into digital data by the A/D converter 11. The blood collection time (T) and the dose (D) of the test substance which correspond to this test substance concentration (Cp) are input using the keyboard 26, thereby to apply these data to the first standard function already obtained in step 105, thereby calculating the clearance (CL).

At this time, a test substance concentration (Cp) obtained using an external hemoanalyzing unit may be input from the keyboard 40 without using the hemoanalyzer 10 and the A/D converter 11.

A detailed technique for calculating the clearance (CL) will be described with reference to a flow chart in Fig. 3. The respective constants Cp, D, T, A, B, $\alpha$, and $\beta$ of the first standard function $Cp/D = A \times \exp(-\alpha \times (CL \times T)) + B \times \exp(-\beta \times (CL \times T))$ are read out from the hard disk 20 (step 110). It is then determined whether $(CL \times T)$ falls within the range (0 to 350 (mℓ/min)·min can be selected as the range of $(CL \times T)$ in the curve shown in, e.g., Fig. 12) of the predetermined upper and lower $(CL \times T)$ limits (step 111).

This determination is performed such that parameters (Cp/D) corresponding to the upper and lower $(CL \times T)$ limits are calculated using the first standard function, and it is determined whether the (Cp/D) values calculated from the input data falls within the range of the above two parameter values. As a result, when it is determined that the $(CL \times T)$ falls outside the range, processing is ended. However, when it is determined that the $(CL \times T)$ falls within the range, the upper and lower $(CL \times T)$ limits are divided into two regions to determine one of the regions to which the (Cp/D) value calculated from the input data belongs (step 112). The two ends of the $(CL \times T)$ region to which the (Cp/D) value belongs are defined as new upper and lower limits.

It is determined whether the range width of the new upper and lower $(CL \times T)$ limits is larger than a predetermined allowance value (i.e., a value determined on the basis of a clearance calculation error) (step 113). In this determination, when the range width of the upper and lower limits is larger than the allowance value, processing returns to step 112 to further divide the range width into two parts. Until the range width of the upper and lower limits becomes the allowance value or less, the processing operations in steps 112 and 113 are repeated to continuously divide the range width into two parts. As a result, when the range width of the upper and lower limits becomes the allowance value or less, one of the (Cp/D) values which has a smaller difference from the (Cp/D) value calculated from the input data is defined as a solution. The clearance (CL) is obtained from the (CL × T) value corresponding to this solution, thereby ending the processing (step 114).

Blood is collected only once in step 106 of FIG. 2, and the load imposed on the test subject is small. This method is particularly suitable for clearance measurements for small animals such as a mouse and a rat. More specifically, when the number of blood collection operations is increased, a small animal such as a mouse or a rat cannot endure the repeated blood collection and often dies. Such a danger can be avoided by the first clearance measuring apparatus 1 capable of measuring the clearance by single blood collection, thereby measuring the clearance safely. After the clearance (CL) is calculated in step 106, the main program 31 outputs the calculation result. This output processing is determined as to whether the output form is a monitor output or a printer output (step 107). When the determination result indicates a monitor output, the calculated clearance (CL) is displayed on the monitor 50 (step 108). When the determination result in step 107 indicates a printer output, the calculated clearance (CL) is output from the printer 60 (step 109).

After the clearance (CL) is calculated in step 106, the calculation result may be stored in, e.g., the hard disk 20 without being output.

Next, a computer program product (clearance measurement storage medium) according to the first embodiment of the present invention will be described hereinbelow.

Fig. 4 is a block diagram showing the data structure of a clearance measurement storage medium 2 according to the first embodiment of the present invention. The clearance measurement storage medium 2 has a program area A which stores a program and a header area B which stores the number of blocks of the program area A, area management data of the program area A, and the like.

The program area A stores a first data accumulation program 32 for accumulating a plurality of measurement data consisting of a dose (D) of a test substance, a blood collection time (T), and a test substance concentration (Cp), and a first standard function formation program 33 for forming a first standard function on the basis of the plurality of measurement data accumulated in the first data accumulation program 32. In addition, the program area A stores a first clearance calculation program (clearance measurement program) 34 for obtaining a clearance (CL) expressed as a flow rate when the excretion amount of a test substance in urine per unit time is converted into a concentration in plasma, and a main program 31 for controlling the programs 32 to 34.

The first clearance calculation program 34 has an input routine 34a for receiving inputs of the dose (D) of the test substance administered to the test subject, the blood collection time (T) required from the administration of the test substance to blood collection with respect to the test subject, and the test substance concentration (Cp) of a plasma sample obtained upon preparing blood collected from the test subject. The first clearance calculation program 34 also has an arithmetic routine 34b for applying the dose (D), the blood collection time (T), and the test substance concentration (Cp) received in the input routine to a first standard function $Cp/D = f_1(CL \times T)$, thereby calculating the clearance (CL).

The first standard function $Cp/D = f_1(CL \times T)$ is a function which takes, as one parameter, the product value of the blood collection time (T) and the clearance (CL) expressed as a flow rate when the excretion amount of a test substance in urine per unit time is converted into a concentration in plasma, and, as the other parameter, a value obtained by dividing the test substance concentration (Cp) by the dose (D). The first standard function $Cp/D = f_1(CL \times T)$ has excellent characteristics; it converges to an almost one type of standard function for each animal species. The first clearance calculation program 34 stored in the clearance measurement storage medium 2 utilizes these characteristics of the first standard function. Therefore, the clearance (CL) can be highly accurately obtained by single administration of the test substance and single blood collection. The characteristics of this first standard function were found by the present inventor for the first time.

The clearance measurement storage medium 2 may be any storage medium capable of optically or magnetically record information, such as a flexible disk, a CD-ROM, or an MD. Examples of the test subject are mammals such as a mouse, a rat, a dog, a monkey, a guinea pig, and a human. In addition, examples of the test substance are inulin and meglumine iothalamate. The first standard function is preferably exemplified as follows:

$$Cp/D = A \times \exp(-\alpha \times (CL \times T))$$

$$+ B \times \exp(-\beta \times (CL \times T))$$

(where A, B, $\alpha$ and $\beta$ are constants specified for each animal species).

The predetermined information processing apparatus reads the programs 31 to 34 stored in the program area A of the clearance measurement storage medium 2, and these programs 31 to 34 can be executed by the information processing apparatus. An example of the information processing apparatus is shown in Figs. 5 and 6. An information processing apparatus 1 shown in Figs. 5 and 6 can be applied to a clearance measurement storage medium 2 according to the second embodiment (to be described later).

Fig. 5 is a block diagram showing the arrangement of the information processing apparatus 1. As shown in Fig. 5, the information processing apparatus 1 comprises a hemoanalyzer 10 for analyzing the test substance concentration (Cp) of a plasma sample obtained upon preparing blood collected from the test subject, an A/D converter 11 for converting the test substance concentration (Cp) as analog data output from the hemoanalyzer 10 into digital data, and a hard disk 20 for storing data such as the test substance concentration (Cp) converted into the digital data and the first standard function data.

The information processing apparatus 1 also comprises a storage medium reader (medium driver) 80 for reading the programs 31 to 34 stored in the clearance measurement storage medium 2, a memory unit 30 for storing the programs 31 to 34 read by the memory medium reader 80, and a keyboard 40 for inputting data such as the dose (D), the blood collection time (T), and a test name (e.g., the name of the test subject and the blood collection date). The information processing apparatus 1 further comprises a monitor 50 for displaying the clearance of a test subject together with input data, a printer 60 for printing out the clearance of the test subject and the like, and a CPU 70 for controlling the hemoanalyzer 10, the hard disk 20, and the storage medium reader 80.

The test substance concentration (Cp) may be detected using an external hemoanalyzing unit, and this test substance concentration (Cp) may be input from the keyboard 40. In this case, the hemoanalyzer 10 and the A/D converter 11 can be omitted. The dose (D), the blood collection time (T), and the test substance concentration (Co) may be externally input through a modem 90 and a communication line (not shown). The processing flow of the programs 31 to 34 upon execution of the programs 31 to 34 stored in the clearance measurement storage medium 2 by the information processing apparatus 1 will be described using a flow chart in Fig. 7. When the clearance measurement storage medium 2 is loaded in the storage medium reader 80, the programs 31 to 34 stored in the program area A of the clearance measurement storage medium 2 are read by the storage medium reader 80 (step 100). The programs 31 to 34 read by the storage medium reader 80 are transferred to the memory unit 30 and stored in it (step 101).

When an operator inputs an operation start instruction using the keyboard 40, the main program 31 stored in the memory unit 30 is activated and, then, the steps 102 to 105 shown in Fig. 7 which are the same as the above-mentioned steps 102 to 105 shown in Fig. 2, respectively, are executed to obtain the first standard function.

When the input instruction is an instruction for selecting the clearance calculation processing in step 103 of the flow chart in Fig. 7, the main program 31 executes the first clearance calculation program 34 stored in the memory unit 30 (step 106). In the first clearance calculation program 34, the input routine 34a is executed to receive the inputs of the dose (D) of the test substance, the blood collection time (T), and the test substance concentration (Cp) (step 106a). More specifically, the test substance is administered to the test subject once, and blood is collected once a predetermined period of time after the administration. Upon execution of the first clearance calculation program 34, the hemoanalyzer 10 detects the test substance concentration (Cp) in each plasma sample upon preparing the collected blood.

The test substance concentration (Cp) thus detected is converted into digital data by the A/D converter 11. The blood collection time (T) and the dose (D) of the test substance which correspond to this test substance concentration (Cp) are input using the keyboard 40, thereby receiving the inputs of the dose (D) of the test substance, the blood collection time (T), and the test substance concentration (Cp).

The arithmetic routine 34b is executed to apply the data input in the input routine 34a to the first standard function already obtained in step 105, thereby calculating the clearance (CL) (step 106b). At this time, a test substance concentration (Cp) obtained using an external hemoanalyzing unit may be input from the keyboard 40 without using the hemoanalyzer 10 and the A/D converter 11.

The clearance (CL) is calculated by means of the above-mentioned steps 110 to 114 in the flow chart shown in Fig. 3.

After the clearance (CL) is calculated in step 106, the main program 31 outputs the calculation result. This output processing is determined as to whether the output form is a monitor output or a printer output (step 107). When the determination result indicates a monitor output, the calculated clearance (CL) is displayed on the monitor 50 (step 108). When the determination result in step 107 indicates a printer output, the calculated clearance (CL) is output from the printer 60 (step 109).

After the clearance (CL) is calculated in step 106, the calculation result may be stored in, e.g., the hard disk 20 without being output.

(Second Embodiment)

A clearance measuring apparatus according to the second embodiment of the present invention will be described below. Fig. 8 is a block diagram showing the arrangement of a clearance measuring apparatus 1 according to the second embodiment. This clearance measuring apparatus 1 is different from the clearance measuring apparatus 1 shown in Fig. 1 in that a second data accumulation program 35, a second standard function formation program 36, and a second clearance calculation program 37 are stored in a memory unit 30 in place of the first data accumulation program 32, the first standard function formation program 33, and the first clearance calculation program 34, respectively.

More specifically, the clearance measuring apparatus 1 is an apparatus for obtaining a clearance (CL) by applying a dose (D), a blood collection time (T), a test substance concentration (Cp), and a steady-state distribution volume (Vss) of a test subject to a second standard function $Cp/(D/Vss) = f_2((CL \times T)/Vss)$. For this purpose, the execution operations of the second data accumulation program 35, the second standard function formation program 36, and the second clearance calculation program 37 are different from those of the clearance measuring apparatus 1 shown in Fig. 1.

The clearance measuring apparatus 1 is an apparatus utilizing the characteristics of the second standard function $Cp/(D/Vss) = f_2((CL \times T)/Vss)$ having excellent characteristics; it converges to an almost one type of standard function for each animal species, thereby highly accurately obtaining the clearance (CL) by single administration of the test substance and single blood collection. The characteristics of the second standard function are the facts found by the present inventor for the first time.

Examples of the test subject are mammals such as a mouse, a rat, a dog, a monkey, a guinea pig, and a human. In addition, examples of the test substance are inulin and meglumine iothalamate. The second standard function is preferably exemplified as follows:

$$Cp/(D/Vss) = A \times \exp(-\alpha \times (CL \times T)/Vss)$$

$$+ B \times \exp(-\beta \times (CL \times T)/Vss)$$

(where A, B, $\alpha$ and $\beta$ are constants specified for each animal species).

The second data accumulation program 35 shown in Fig. 8 is a program in which a plurality of measurement data consisting of the dose (D), the blood collection time (T), the test substance concentration (Cp), and the steady-state distribution volume (Vss) are stored in a hard disk 20. The second standard function formation program 36 is a program for forming a second standard function on the basis of the plurality of measurement data stored in the hard disk 20. In addition, the second clearance calculation program 37 is a program for calculating the clearance (CL) by applying the dose (D), the blood collection time (T), the test substance concentration (Cp), and the steady-state distribution volume (Vss) to the second standard function.

Note that the steady-state distribution volume (Vss) can be obtained by substituting the body weight (BW) of a mammal as a subject for clearance measurement into the following equation:

$$Vss = BW \times Ks$$

where Ks is the conversion coefficient for converting the weight into the steady-state distribution volume (Vss). In this case, assume the density of a mammal is defined as 1 $g/cm^3$. The conversion coefficient (Ks) varies depending on different animal species and is experimentally obtained. More specifically, the steady-state distribution volumes (Vss) of a maximum number of individuals are obtained and divided by the weights (BW) of the respective individuals, and the quotients are averaged to obtain the conversion coefficient (Ks). For example, in Examples 11 and 12 to be described later, the conversion coefficient (Ks) for a rat is said to be 0.260, the conversion coefficient (Ks) for a dog is said to be 0.176, and the conversion coefficient (Ks) for a human is said to be 0.2.

Next, a clearance measuring method according to the second embodiment of the present invention using the clearance measuring apparatus 1 will be described with reference to a flow chart in Fig. 9. When an operator inputs an operation start instruction using the keyboard 40, the main program 31 is activated. In the main program 31, a selection menu for selecting execution of one of second standard function formation processing and clearance calculation processing is displayed on the monitor 50 (step 122). When the operator inputs a processing selection instruction for selecting one of the above processing operations using the keyboard 40, it is determined whether the input represents one of the processing operations (step 123). When the input instruction is an instruction for selecting the second standard function formation processing, the main program 31 executes the second data accumulation program 35 (step 124).

Upon execution of the second data accumulation program 35, n sets of measurement data consisting of a dose (D) of a test subject, blood collection times ($T_i$, i = 1 to n), test substance concentrations ($Cp_i$, i = 1 to n), and steady-state distribution volume (Vss) are stored in the hard disk 20. More specifically, the test substance is subjected to single intravenous injection to the test subject, and blood is collected four or more times normally during 4 to 8 hours after the single intravenous injection. When the second data accumulation program 35 is executed, the hemoanalyzer 10 measures the target substance concentration ($Cp_i$) of each plasma sample obtained upon preparing the collected blood.

Each test substance concentration ($Cp_i$) thus measured is converted into digital data by the A/D converter 11. When the blood collection time ($T_i$), the dose (D) of the test substance, and the steady-state distribution volume (Vss) which correspond to this test substance concentration ($Cp_i$) are input using the keyboard 40, the resultant data are stored as measurement data in the hard disk 20. These measurement data are obtained for each blood collection, so that a plurality of sets of measurement data are stored in the hard disk 20. In this case, test substance concentrations ($Cp_i$) obtained using an external hemoanalyzing unit may be input from the keyboard 40 without using the hemoanalyzer 10 and the A/D converter 11.

When execution of the second data accumulation program 35 is completed, the main program 31 causes to execute the second standard function formation program 36 (step 125). Upon execution of the second standard function formation program 36, the clearance (CL) is obtained from the plurality of sets of measurement data stored in the hard disk 20, thereby forming a second standard function.

It is preferable to perform calculation processing of the clearance (CL) for a maximum number of specimens having different blood collection times of each individual. In addition, it is also preferable to perform calculation processing of the clearance (CL) for a maximum number of individuals belonging to each animal species. The constants A, B, $\alpha$ and $\beta$ of the second standard function $Cp/(D/Vss) = A \times \exp(-\alpha \times (CL \times T)/Vss) + B \times \exp(-\beta \times (CL \times T)/Vss)$ are calculated using the method of least squares so as to minimize the errors on the basis of the resultant clearances (CL) of each individual, and the blood collection times ($T_i$), the test substance concentrations ($Cp_i$), the dose (D), and the steady-state distribution volume (Vss) which correspond to these clearances (CL), thereby obtaining the second standard function.

When the input instruction is an instruction for selecting the clearance calculation processing in step 123, the main program 31 executes the second clearance calculation program 37 (step 126). A clearance of the test substance can be calculated by the second clearance calculation program 37. More specifically, the test substance is administered to the test subject once, and blood is collected once a predetermined period of time after the administration. Upon execution of the second clearance calculation program 37, the hemoanalyzer 10 detects the test substance concentration (Cp) in each plasma sample upon preparing the collected blood.

The test substance concentration (Cp) thus detected is converted into digital data by the A/D converter 11. When the blood collection time (T), the dose (D) of the test substance, and the steady-state distribution volume (Vss) which correspond to this test substance concentration (Cp) are input using the keyboard 40, thereby to apply these data to the second standard function already obtained in step 125, thereby calculating the clearance (CL). At this time, a test substance concentration (Cp) obtained using an external hemoanalyzing unit may be input from the keyboard 40 without using the hemoanalyzer 10 and the A/D converter 11.

After the clearance (CL) is calculated, the main program 31 outputs the calculation result. This output processing is determined as to whether the output form is a monitor output or a printer output (step 127). When the determination result indicates a monitor output, the calculated clearance (CL) is displayed on the monitor 50 (step 128). When the determination result in step 127 indicates a printer output, the calculated clearance (CL) is output from the printer 60 (step 129).

After the clearance (CL) is calculated in step 126, the calculation result may be stored in, e.g., the hard disk 20 without being output.

Next, a computer program product (clearance measurement storage medium) according to the second embodiment of the present invention will be described hereinbelow.

Fig. 10 is a block diagram showing the arrangement of a clearance measurement storage medium 2 of the second embodiment. The clearance measurement storage medium 2 has a program area A which stores a program and a header area B which stores the number of blocks of the program area A, area management data of the program area A, and the like.

The program area A stores a second data accumulation program 35 for accumulating a plurality of measurement data consisting of a dose (D) of a test substance, a blood collection time ($T_i$), a test substance concentration ($Cp_i$), and a steady-state distribution volume (Vss), and a second standard function formation program 36 for forming a second standard function on the basis of the plurality of measurement data accumulated in the second data accumulation program 35. In addition, the program area A stores a second clearance calculation program (clearance measurement program) 37 for obtaining a clearance (CL) as an excretion amount of the test substance in urine per unit time, and a main program 31 for controlling the programs 35 to 37.

The second clearance calculation program 37 has an input routine 37a for receiving inputs of the dose (D) of the test substance administered to the test subject, the blood collection time (T) required from the administration of the test substance to blood collection with respect to the test subject, the test substance concentration (Cp) of a plasma sample obtained upon preparing blood collected from the test subject, and the steady-state distribution volume (Vss) of the test subject. The second clearance calculation program 37 also has an arithmetic routine 37b for applying the dose (D), the blood collection time (T), the test substance concentration (Cp), and the steady-state distribution volume (Vss) received in the input routine to a second standard function $Cp/(D/Vss) = f_2((CL \times T)/Vss)$, thereby calculating the clearance (CL).

The second standard function $Cp/(D/Vss) = f_2((CL \times T)/Vss)$ is a function which takes, as one parameter, a value obtained by dividing the product value of the blood collection time (T) and the clearance (CL) expressed as a flow rate when the excretion amount of a test substance in urine per unit time is converted into a concentration in plasma by the steady-state distribution volume (Vss), and, as the other parameter, a value obtained by dividing the test substance concentration (Cp) by the ratio of the dose (D) to the steady-state distribution volume (Vss). The second standard function $Cp/(D/Vss) = f_2((CL \times T)/Vss)$ has excellent characteristics; it converges to an almost one type of standard function for each animal species as in the first standard function. The second clearance calculation program 37 stored in the clearance measurement storage medium 2 utilizes these characteristics of the second standard function. Therefore, the clearance (CL) can be highly accurately obtained by single administration of the test substance and single blood collection. The characteristics of this second standard function were found by the present inventor for the first time.

The clearance measurement storage medium 2 may be any storage medium capable of optically or magnetically record information, such as a flexible disk, a CD-ROM, or an MD. Examples of the test subject are mammals such as a mouse, a rat, a dog, a monkey, a guinea pig, and a human. In addition, examples of the test substance are inulin and meglumine iothalamate. The second standard function is preferably exemplified as follows:

$$Cp/(D/Vss) = A \times \exp(-\alpha \times (CL \times T)/Vss)$$

$$+ B \times \exp(-\beta \times (CL \times T)/Vss)$$

(where A, B, $\alpha$ and $\beta$ are constants specified for each animal species).

Note that the steady-state distribution volume (Vss) can be obtained by substituting the weight (BW) of a mammal as a subject for clearance measurement into the following equation:

$$Vss = BW \times Ks$$

where Ks is the conversion coefficient for converting the weight into the steady-state distribution volume (Vss). In this case, assume the density of a mammal is defined as 1 g/cm$^3$. The conversion coefficient (Ks) varies depending on different animal species and is experimentally obtained. More specifically, the steady-state distribution volumes (Vss) of a maximum number of individuals are obtained and divided by the body weights (BW) of the respective individuals, and the quotients are averaged to obtain the conversion coefficient (Ks). For example, in Examples 11 and 12 to be described later, the conversion coefficient (Ks) for a rat is said to be 0.260, the conversion coefficient (Ks) for a dog is said to be 0.176, and the conversion coefficient (Ks) for a human is said to be 0.2.

The processing flow of the programs 31 and 35 to 37 upon execution of the programs 31 and 35 to 37 stored in the clearance measurement storage medium 2 by the information processing apparatus 1 in Fig. 5 will be described using a flow chart in Fig. 11. When the clearance measurement storage medium 2 is loaded in the storage medium reader 80, the programs 31 and 35 to 37 stored in the program area A of the clearance measurement storage medium 2 are read by the storage medium reader 80 (step 120). The programs 31 and 35 to 37 read by the storage medium reader 80 are transferred to the memory unit 30 and stored in it (step 121).

When an operator inputs an operation start instruction using the keyboard 40, the main program 31 stored in the memory unit 30 is activated and, then, the steps 122 to 125 shown in Fig. 11 which are the same as the above-mentioned steps 122 to 125 shown in Fig. 9, respectively, are executed to obtain the second standard function.

When the input instruction is an instruction for selecting the clearance calculation processing in step 123, the main program 31 executes the second clearance calculation program 37 (step 126). In the second clearance calculation program 37, the input routine 37a is executed to receive the inputs of the dose (D) of the test substance, the blood collection time (T), the test substance concentration (Cp), and the steady-state distribution volume (Vss) (step 126a). More specifically, the test substance is administered to the test subject once, and blood is collected once a predetermined period of time after the administration. Upon execution of the second clearance calculation program 37, the hemoanalyzer 10 detects the test substance concentration (Cp) in each plasma sample upon preparing the collected blood.

The test substance concentration (Cp) thus detected is converted into digital data by the A/D converter 11. When the blood collection time (T), the dose (D) of the test substance, and the steady-state distribution volume (Vss) which correspond to this test substance concentration (Cp) are input using the keyboard 40, thereby receiving the inputs of the dose (D) of the test substance, the blood collection time (T), the test substance concentration (Cp), and the steady-state distribution volume (Vss).

The arithmetic routine 37b is executed to apply the data input in the input routine 37a to the second standard function already obtained in step 125, thereby calculating the clearance (CL) (step 126b). At this time, a test substance concentration (Cp) obtained using an external hemoanalyzing unit may be input from the keyboard 40 without using the hemoanalyzer 10 and the A/D converter 11.

The clearance (CL) is calculated by means of the above-mentioned steps 110 to 114 in the flow chart shown in Fig. 3.

After the clearance (CL) is calculated, the main program 31 outputs the calculation result. This output processing is determined as to whether the output form is a monitor output or a printer output (step 127). When the determination result indicates a monitor output, the calculated clearance (CL) is displayed on the monitor 50 (step 128). When the determination result in step 127 indicates a printer output, the calculated clearance (CL) is output from the printer 60 (step 129).

After the clearance (CL) is calculated in step 126, the calculation result may be stored in, e.g., the hard disk 20 without being output.

In the meanwhile, when the renal function becomes insufficient, the clearance is reduced. The clearance measuring apparatus, method and computer program product of the present invention are effective to diagnose various diseases causing insufficiency of the renal function, such as renal diseases and other abnormalities. Examples of a variety of diseases causing a decrease in clearance and other abnormalities are acute renal insufficiency, chronic renal insufficiency, nephrosclerosis (hypertension), pyelonephritis, acute glomerulonephritis, multiple myeloma, sodium deficiency, shock, apoplexy, norepinephrine, and enuresis.

As described above, the first and second standard functions allow highly accurate calculation of clearances upon single blood collection when the test substance concentration in plasma obtained from a collected blood sample is applied, provided that the first and second standard functions are obtained for each animal species. Note that it is more convenient to improve the clearance calculation accuracy when the samples of each animal species are classified in accordance with the age and sex to finely classify the respective standard functions.

Next, the first and second standard curves of the present invention will be described hereinbelow.

When the first or second standard curve of the present invention is used, for example, a test substance is administered to a test subject at a known dose (D), a test substance concentration (Cp) in a plasma sample prepared upon blood collection is measured, and a clearance (CL), i.e., a renal clearance (CL) can be calculated from the first standard curve using a ratio (Cp/D) of the test substance concentration (Cp) in the plasma sample to the dose (D) of the test substance, and a time (T) required from the administration to blood collection.

In this case, after the test substance is administered once, only the test substance concentration (Cp) of a plasma sample obtained upon single blood collection at arbitrary time is used to highly accurately decide the clearance (CL).

Furthermore, the first standard curve can be used in a new test substance clearance measuring method consisting of the following steps:

(1) the step of administering the test substance at the known dose (D) to the test subject;
(2) the step of collecting blood from the test subject to prepare a plasma sample and measuring the test substance concentration (Cp) in the plasma sample;
(3) the step of calculating a ratio (Cp/D) of the test substance concentration (Cp) obtained in the step (2) to the test substance dose (D) in the step (1);
(4) the step of reading the value of the product ($CL \times T$) of the clearance (CL) and the blood collection time (T) required from the administration to blood collection, from the preformed first standard curve using the ratio (Cp/D) obtained in the step (3); and
(5) the step of dividing the value ($CL \times T$) obtained in the step (4) by the blood collection time (T) to calculate the clearance (CL).

In this case, after the test substance is administered once, the clearance (CL) can be highly accurately decided using only the test substance concentration (Cp) associated with the plasma sample obtained upon single blood collection at arbitrary time.

In addition, the second standard curve can be used for a new test substance clearance measuring method consisting of the following steps:

(1) the step of administering the test substance to the test subject at the known dose (D);
(2) the step of collecting blood from the test subject to prepare a plasma sample and measuring the test substance concentration (Cp) in the plasma sample;
(3) the step of calculating a value [Cp/(D/Vss)] obtained by dividing the test substance concentration (Cp) by a ratio (D/Vss) of the test substance dose (D) in the step (1) to the steady-state distribution volume (Vss);

(4) the step of reading a value [(CL × T)/Vss] obtained by dividing the value of the product (CL × T) of the clearance (CL) and the blood collection time (T) required from the administration to blood collection by the steady-state distribution volume (Vss), from the preformed second standard curve, using the value [Cp/(D/Vss)] obtained in the step (3); and

(5) the step of dividing the value [(CL × T)/Vss] obtained in the step (4) by a ratio (T/Vss) of the blood collection time (T) to the steady-state distribution volume (Vss) to calculate the clearance (CL).

In this case, after the test substance is administered once, the clearance (CL) can be highly accurately decided using only the test substance concentration (Cp) associated with the plasma sample obtained upon single blood collection at arbitrary time.

Each above-mentioned measuring method using the first or second standard curve can preferably be used for clearance measurements of mammals (except for humans) such as a pet (e.g., a dog and a cat) and an experimental animal (e.g., a mouse, a rat, a dog, a monkey, and a guinea pig). In particular, when the number of blood collection operations is increased, a small animal such as a mouse or a rat cannot endure the repeated blood collection and often dies. The method capable of performing one clearance measurement by single blood collection is particularly preferable in view of a reduction in load imposed on small animals. More specifically, this method can be used to diagnose the renal function of a pet as in the human. One clearance measurement for an experimental animal can be performed by single blood collection. The renal function of each individual can be evaluated for a longer period of time than that of the conventional method. For this reason, this method is effective for high-precision drug effect evaluation of a renal disease compound and the like, high-precision screening, and high-precision evaluation of side-effect of each drug to the renal function. In addition, the method is also useful to grasp a change in renal function of an animal, which is caused by the environment and aging as in the human.

The test substances used in the present invention are not limited to specific ones, if they are not susceptible to metabolism in a living body and are subjected to excretion through glomerular filtration; or they are not secreted or reabsorbed by uriniferous tubules; or they are not subjected to metabolism in a living body and are subjected to excretion through glomeruli and uriniferous tubules. Examples of such a substance are inulin, meglumine iothalamate, sodium para-aminohippurate, $^{123}$I-orthoiodohippurate (OIH), $^{131}$I-OIH, $^{99m}$Tc-mercaptoacetylglycylglycylglycine (mercaptoacetyltriglycine: $MAG_3$), sodium thiosulfate, Diodrast, $^{125}$I-iothalamate, $^{99m}$Tc-diethylenetriaminepentaacetate (DTPA), and $^{51}$Cr-ethylenediaminetetraacetate (EDTA). Inulin, meglumine iothalamate, sodium para-aminohippurate, sodium thiosulfate, or Diodrast is preferable. Inulin, meglumine iothalamate, or sodium para-aminohippurate is more preferable. Inulin or meglumine iothalamate is most preferable. Among them all, inulin is particularly preferable.

The inulin used in the present invention may be extracted from natural plants such as the rootstock of a dahlia, a Jerusalem artichoke (Helianthus tuberosus), or a chicory (Chicorium intybus), the fresh rootstock of a sea onion, or seaweeds of Dasycladales; or inulin commercially available as a reagent. Inutest (tradename) (Polyfructosan) can be used equally as inulin. Inutest can be accessible from Laevosan G.m.b.H. as Inutest-Ampullen (Polyfructosan content: 5.0 g).

Meglumine iothalamate (i.e., N-methylglucamine-5-acetamido-2,4,6-triiodo-N-methylisophthalate) is known as a contrast medium. For example, a conray injection solution (60 W/V%) (meglumine iothalamate injection solution of the Japanese Pharmacopoeia) is available from DAIICHI PHARMACEUTICAL CO., LTD.

Sodium para-aminohippurate is available as an injection solution from DAIICHI PHARMACEUTICAL CO., LTD.

$^{123}$I-IOH and $^{131}$I-IOH are markers using Hippuran (orthoiodohippurate; OIH). $^{123}$I-OIH is available from, e.g., Nihon Mediphysics, and $^{131}$I-OIH is available from Daiichi Radioisotope Kenkyujo.

$^{99m}$Tc-$MAG_3$ is a substance obtained by marking mercaptoacetylglycylglycylglycine (mercapthacetyltriglycine) with $^{99m}$Tc. For example, this substance is available from Marine Clott, U.S.A. as Techne Scan $MAG_3$.

Sodium thiosulfate is available from Banyu Pharmaceutical Co., Ltd. as Detoxisole which is a kind of antidote for heavy metal intoxication.

Diodrast is an iodine compound derived from pyridine.

$^{99m}$Tc-DTPA is a substance obtained by marking diethylenetriaminepentaacetate (DTPA) with $^{99m}$Tc. For example, this substance is available from Daiichi Radioisotope Kenkyujo as a TECNE DTPA kit.

$^{51}$cr-EDTA is a substance obtained by marking ethylenediaminetetraacetate (EDTA) with $^{51}$Cr.

The first and second standard curves of the present invention are normally used in measurement of clearances of mammals and preferably in the clearance measurement of mouses, rats, dogs, monkeys, guinea pigs, or humans.

When the first and second standard curves of the present invention are used, the concentration of a test substance in plasma of each sample is measured to allow clearance measurement. One sample here means plasma obtained upon single blood collection at arbitrary time upon single administration of the test substance.

Single administration of the test substance is generally intravenous injection. This single administration may be drip or injection depending on the dose (D) of the test substance. The dose (D) varies depending on the type of test substance and the animal species and is selected in accordance with a given situation. When inulin is used as the test substance, the dose (D) is, e.g., normally 30 to 300 mg/kg for a rat, normally 5 to 200 mg/kg for a dog, and 10 to 200 mg/kg for a human. When meglumine iothalamate is used as the test substance, the dose (D) is, e.g., normally 30 to 300 mg/kg for a rat, 5 to 200 mg/kg for a dog, and 10 to 200 mg/kg for a human.

The blood collection portion may be a vein or artery, and is preferably a vein. The blood collection time (T) required from administration to blood collection is not limited to a specific one, but normally falls within the range of 0.1 to 5 hours, and preferably 0.5 to 2 hours. The blood collection amount varies depending on the animal species and the doses (D). Appropriate blood collection amounts are selected depending on the respective situations. For example, when inulin is used as the test substance, the blood collection amount is, e.g., normally 0.1 to 2 mℓ and preferably 0.5 to 1 mℓ for a rat, normally 0.5 to 5 mℓ and preferably 0.5 to 2 mℓ for a dog, and 0.5 to 5 mℓ and preferably 0.5 to 2 mℓ for a human. When meglumine iothalamate is used as the test substance, the blood collection amount is, e.g., normally 0.1 to 2 mℓ and preferably 0.5 to 1 mℓ for a rat, normally 0.5 to 5 mℓ and preferably 0.5 to 2 mℓ for a dog, and 0.5 to 5 mℓ and preferably 0.5 to 2 mℓ for a human.

In measurement of the test substance concentration (Cp) in the hemoanalyzer 10, an appropriate measurement method is employed for each test substance. More specifically, for example, in measurement of the inulin concentration in plasma, the following measurement method is used. Inulin is a polymer compound (polyfructosan) having a molecular weight of about 5,000 and has a wide molecular weight distribution. It is therefore difficult to analyze the inulin as it is. Inulin as a polyfructosan is subjected to acidic hydrolysis to convert it into a fructose, and the resultant fructose is quantitatively measured by a variety of methods as follows.

Examples of the fructose quantitative measurement method are (A) measurement methods using a high-speed liquid chromatography and a pulse type electrochemical detector (Akiyoshi Soga, Gekkan Food Chemical, 1991, December edition, pp. 44 - 48), (B) enzymatic measurement methods (Japanese Patent Laid-Open No. 62-205799, H.F. Kuenle et al., Nephron, Vol. 62, pp. 104 - 107, 1992, EP 0021310A1, D.F. Day et al., Ann. N.Y. Acad. Sci. (USA), Vol. 434, pp. 504 - 507, 1984), and (C) color development methods (H.D. Harrison, Proc. Soc. Exp. Biol. Med., Vol. 49, pp. 111 - 114, 1942, G.F. Schreiner, Proc. Soc. Exp. Biol. Med., Vol. 74, pp. 117 - 120, 1950, and W.D. Davidson et al., J. Lab. Clin. Med., Vol. 62, pp. 351 - 356, 1963). In this embodiment, method (A) or (B) is preferably used, and method (A) is more preferable because it is excellent in sensitivity and qualitative measurement.

A new inulin measurement method (Japanese Patent Application No. Hei 7-53297 (53297/95)) proposed by the present inventor is particularly preferably used in the measurement of the inulin concentration in plasma. According to this method, glucose and fructose are removed from the plasma through a membrane, and acidic hydrolysis is performed to convert the inulin into fructose, and the resultant fructose is quantitatively measured. Note that the anthrone method using anthrone as a color developing agent is a conventionally popular inulin quantitative measurement method.

In measurement of the meglumine iothalamate in plasma, a method of adding 400 μℓ of a solution mixture of 0.1N HCl and methanol (1 : 9) to 100 μℓ of plasma and stirring the resultant solution well, and analyzing the supernatant obtained upon centrifugal separation, using liquid chromatography, is used. In addition, in measurement of the sodium para-aminohippurate concentration in plasma, after proteins are removed, the supernatant is analyzed by liquid chromatography. In measurement of the $^{123}$I-OIH, $^{131}$I-OIH, $^{99m}$Tc-MAG$_3$, $^{125}$I-iothalamate, $^{99m}$Tc-DTPA, and $^{51}$Cr-EDTA concentrations in plasma, for example, a method of measuring these concentrations using a gamma counter is used. In measurement of the sodium thiosulfate or Diodrast concentration in plasma, after proteins are removed, titrimetry of potassium iodide using starch as an indicator is used.

A method of forming the first and second standard curves according to the present invention will be described below.

The following fact is found by the present inventors for the first time. That is, clearances (CL) are calculated from the two-compartment model using test substance concentrations (Cp) in plasma of blood collected at the respective times (T) upon single test substance intravenous injection (dose D) to a large number of mammals ranging from healthy mammals having the normal renal function to mammals having renal diseases of various levels. Using these clearance (CL) values, the product of CL and T is plotted along one of the coordinate axes, and a ratio value of Cp to D is plotted along the other of the coordinate axes. Surprisingly, the plotted points converge to one curve (CL × T - Cp/D curve) for each animal species.

The following fact is also found by the present inventors for the first time. Using a steady-state distribution volume (or total distribution volume) (Vss) obtained from the two-compartment model, CL × T/Vss is plotted along one of the coordinate axes, while Cp/(D/Vss) is plotted along the other of the coordinate axes. In this case, the plotted points converge to one curve [CL × T/Vss - Cp/(D/Vss) curve] for each animal species.

(a) Method of Obtaining Clearance Using Two-compartment Model (Conventional Method)

After a test substance is administered by single intravenous injection, blood collection is normally performed at four or more time points during 4 to 6 hours after the intravenous injection, and the measured test substance concentrations (Cp) in plasma are plotted as a function of time (T).

An amount of test substance (dU) excreted in urine per unit time can be expressed as the product (dU = CL × Cp) of the test substance concentration (Cp) in plasma and the clearance (CL). From this dU = CL × Cp, the following equation can be derived:

$$\int_0^{\infty} dUdT = CL \times \int_0^{\infty} CpdT$$

since the left-hand side, that is,

$$\int_0^{\infty} dUdT$$

is the total amount of the test substance excreted in urine and is equal to the test substance dose (D).

$$\int_0^{\infty} CpdT$$

becomes the area (AUC) under the curve of the test substance concentration in plasma. Therefore,

$$\int_0^{\infty} dUdT = CL \times \int_0^{\infty} CpdT$$

can be rewritten as D = CL × AUC, so that the clearance is given as follows:

$$CL = D/AUC.$$

The curve obtained by plotting the test substance concentration (Cp) in plasma as a function of time (T) can be approximated to a two-compartment model [$Cp = M \times \exp(-\mu T) + N \times \exp(-\nu T)$] consisting of the first phase as the distribution phase and the second phase as the excretion phase. This approximated curve is estimated to obtain AUC, thereby calculating CL as D/AUC.

The formation of the approximated curve and the calculation of the CL value are performed using a technique described in, e.g., a reference "Kiyoshi Yamaoka, Dynamic Analysis Method of Drugs in Body Using Microcomputer, pp. 100 - 105, Nanko-do (1984)".

The two-compartment model will be briefly described from the Yamaoka's reference. The administered test material enters into a portion (compartment 1) regarded to be almost equivalent to the circulation system of the body and reaches an equivalent state. The test material then shifts (velocity constant $k_{12}$) to a portion (compartment 2) which is not equivalent to the circulation system of the body, shifts (velocity constant $k_{21}$) from compartment 2 to compartment 1, and disappears (velocity constant $k_e$) from compartment 1. Therefore, the disappearance formula of the test material from compartments 1 and 2 is solved to derive an equation $Cp = M \times \exp(-\mu T) + N \times \exp(-\nu T)$ where M, N, $\mu$, and $\nu$ are constants. The distribution volume (V1) of compartment 1 is defined as V1 = D/(A + B). Similarly, the distribution volume (V2) of compartment 2 is defined as $V2 = V1 \times k_{12}/k_{21}$. The steady-state distribution volume (Vss) can be calculated by Vss = V1 + V2. Furthermore, $AUC = M/\mu + N/\nu$. All these parameters of each individual are calculated by the computer software.

(b) Formation of First Standard Curve [CL × T - Cp/D curve] (Present Invention)

For each individual used in item (a), a value obtained by multiplying CL ($m\ell$/min) obtained in item (a) by T (min) is plotted along the abscissa, and a value obtained by dividing Cp (mg/d$\ell$) corresponding to T (min) by D (mg) is plotted along the ordinate. With this arrangement, the abscissa has a dimension of [mℓ], and the ordinate has a dimension of [1/dℓ], which is equivalent almost nondimensional conversion. The ordinate and abscissa may be reversed.

This plot is a two-phase curve similar to the T - (Cp) curve obtained in item (a). For this reason, computer software for obtaining an approximated curve of the two-compartment model can be used to obtain an approximated curve.

In addition, an example of the computer software useful to obtain an approximated curve is SCIENTIST (TM) available from Micromath.

The present inventors have found that the resultant CL × T - Cp/D curve converges to one type of standard curve for each animal species. When the first standard curve [CL × T - Cp/D curve] is formed for each animal species in advance, a clearance can be found to be calculated as follows. After a test substance is administered once to each test subject animal, a test substance concentration (Cp) in plasma of one sample obtained by blood collection an arbitrary period of time after the administration is obtained. The clearance can be calculated from the test substance concentration (Cp) using the above first standard curve.

More specifically, the value of the ratio of the test substance concentration (Cp) in plasma to the test substance dose (D) is calculated, and the product of the clearance (CL) and the blood collection time (T) can be obtained using the above ratio value. The final product is divided by the blood collection time (T), thereby calculating the clearance (CL).

(c) Formation of Second Standard Curve [CL × T/Vss - Cp/(D/Vss) Curve] (Present Invention)

A value obtained by dividing the product of CL (mℓ/min) obtained in item (a) and T (min) by Vss (mℓ) is plotted along the abscissa, and a value obtained by dividing Cp (mg/dℓ) corresponding to T (min) by a ratio value of D (mg) to Vss (dℓ) is plotted along the ordinate. With this arrangement, the abscissa and ordinate are nondimensional. The ordinate and the abscissa may be reversed.

This plot is a two-phase curve similar to T - (Cp) obtained in item (a). An approximated curve can be obtained as in the first standard curve.

The present inventors have found that the resultant CL × T/Vss - Cp/(D/Vss) curve also converges to one type of standard curve for each animal species. When the second standard curve [CL × T/Vss - Cp/(D/Vss) curve] is formed for each animal species, a clearance is found to be calculated as follows. After a test substance is administered once to each test subject animal, the test substance concentration (Cp) in plasma of each sample derived from blood collection an arbitrary period of time after the administration, and the steady-state distribution volume (Vss) are obtained. Using the second standard curve, the clearance can be calculated from the test substance concentration (Cp) in plasma and the steady-state distribution volume (Vss).

The steady-state distribution volume Vss in single blood collection can be estimated by the following equation using the body weight (BW) of a mammal as a subject for clearance measurement:

$$Vss = BW \times Ks$$

where Ks is the conversion coefficient for converting the body weight into the steady-state distribution volume (Vss). In this case, assume the density of a mammal is defined as 1 g/cm$^3$. The conversion coefficient (Ks) varies depending on different animal species and is experimentally obtained. The Vss values of the respective individuals are obtained from the Cp-T curve of a large number of individuals and divided by the weights BW of the respective individuals to obtain an average value as Ks. For example, within the scope of the embodiments of the present invention, Ks for a rat is obtained as 0.260, and Ks for a dog is obtained as 0.176 (see Examples 10 and 11). Note that Ks for a human is said to be 0.2.

To calculate a clearance using the second standard curve [CL × T/Vss - Cp/(D/Vss) curve], the test substance concentration (Cp) in plasma is divided by the ratio value of the test substance dose (D) to the Vss value calculated in the above formula to obtain Cp/(D/Vss). Using this value, the CL × T/Vss value is read from the second standard curve. The read value is multiplied by Vss/T to obtain the clearance (CL).

According to the present invention, the method using the first standard curve [CL × T - Cp/D curve] and the method using the second standard curve [CL × T/Vss - Cp/(D/Vss) curve] can be selectively used, but the latter curve is preferable because it is nondimensional.

[Examples]

<u>Example 1: Formation of First Standard Function [CL × T - Cp/D Curve] for Rat</u>

A first standard function was formed upon execution of the first data accumulation program 32 and the first standard function formation program 33.

As rats, female SD rats (body weight: 220 to 340 g) were used. A total of fifteen rats consisting of three healthy rats (rat Nos. N-1 to N-3), two unilaterally nephrectomized rats (rat Nos. Uni-1 and Uni-2), and 10 partially nephrectomized rats (rat Nos. CRF-1 to CRF-10) were used.

Nembutal (45 mg/kg) was administered in the abdominal cavities of the rats to anesthetize them. The prospective incision portions of the right and left jugular veins of the anesthetized rats were shaved, and the samples were fixed onto a 37°C plate. The tracheae of the rats were incised and polyethylene tubes were inserted into the tracheae of the respective rats to prevent respiratory insufficiency. Blood (0.2 mℓ) was collected from the left jugular vein of each rat. To estimate the morbid state, the serum creatinine value (Cr) and blood urea nitrogen value (BUN) of each rat were measured. The Cr and BUN of the respective rats are summarized in Table 1.

[Table 1]

| Rat. No. | Cr (mg/dℓ) | BUN (mg/dℓ) |
|---|---|---|
| N-1 | 0.4 | 14 |
| N-2 | 0.4 | 10 |
| N-3 | 0.4 | 20 |
| Uni-1 | 0.4 | 24 |
| Uni-2 | 0.4 | 20 |
| CRF-1 | 1.7 | 83 |
| CRF-2 | 2.6 | 110 |
| CRF-3 | 3.3 | 79 |
| CRF-4 | 2.0 | 58 |
| CRF-5 | 0.8 | 52 |
| CRF-6 | 1.0 | 65 |
| CRF-7 | 0.6 | 32 |
| CRF-8 | 1.2 | 37 |
| CRF-9 | 1.7 | 67 |
| CRF-10 | 1.4 | 57 |

A 10% inulin solution (0.5 mℓ, 50 mg, USP-manufactured in the U.S.A.) was administered in the right jugular vein of each rat using a 1-mℓ syringe. The blood collection time slightly varied depending on the renal insufficiency states. Five blood collection operations (0.2 mℓ each) were performed in three hours after the administration, and the plasma was immediately separated from the blood each time. Typical examples of the blood collection time were 5 min, 30 min, 60 min, 120 min, and 180 min after the administration.

An external hemoanalyzing unit was used to measure the inulin concentration in plasma as follows. The plasma was dialyzed through a regenerated cellulose membrane having a fractional molecular weight of 1,000, 5% tricholoroacetate was added to the dialyzed plasma, and the proteins were removed from the resultant solution by centrifugal separation, thereby obtaining a supernatant. 12N sulfuric acid was added to the supernatant (final concentration of about 1N), and the resultant solution was hydrolyzed at 60°C for 5 hours. Solid barium carbonate was added to the hydrolyzed solution to neutralize it. The fructose content in each neutralized sample was measured by a high-speed liquid chromatograph/pulse type electrochemical detection system (BIO·LC available from DIONEX) to calculate the inulin concentration in plasma.

The resultant inulin concentrations (Cp) in plasma were plotted as a function of the blood collection times (T) to estimate an approximated curve of the two-compartment model in accordance with the Yamaoka's reference (Kiyoshi Yamaoka, Dynamic Analysis Method of Drugs in Body Using Microcomputer, pp. 100 - 105, Nanko-do (1984)). The area (AUC) under the curve was obtained, and the inulin clearance (CL) was calculated as follows (dose (D) = 50mg):

$$CL = D/AUC.$$

A value obtained by multiplying the resultant CL (mℓ/min) with T (min) was plotted along the abscissa, and a value obtained by dividing Cp (mg/dℓ) corresponding to this T (min) by D (mg) was plotted along the ordinate. These points are apparently approximated by one curve from Fig. 12. Note that A, B, α and β in Fig. 12 (and Figs. 13 to 18) are constants, and E±n represents

$10^{\pm n}$

The information processing apparatus 1 was activated to input the inulin concentrations ($Cp_i$) in the plasma samples obtained by the external hemoanalyzing unit, the blood collection times ($T_i$), and the dose (D) using the keyboard 40. An inulin clearance (CL) was calculated using the above-mentioned AUC method. These data were applied to Cp/D = A × exp(-α × (CL × T)) + B × exp(-β × (CL × T)) to calculate the constants A, B, α and β, thereby obtaining a first standard function. This first standard function is shown in Fig. 12.

Example 2: Formation of First Standard Function [CL × T - Cp/D Curve] for Dog

A first standard function was formed upon execution of the first data accumulation program 32 and the first standard function formation program 33.

A total of five female beagle dogs (body weight: 8.5 to 14 kg) consisting of one healthy dog (dog No. 1919), three partially nephrectomized dogs (chronic renal failure model) (dog Nos. 1367, 1253, and 1104) and unilaterally nephrectomized dog (dog No. 963) were used.

A 3% inulin-containing iothalamate solution was administered once in the amount of 0.3 mℓ/kg to each beagle dog from its foreleg vein. Blood collection operations were performed five times with intervals after the administration. Following the same procedures as in Example 1, the constants A, B, α and β were calculated to form a first standard function. The blood collection times were, e.g., 5 min, 20 min, 40 min, 60 min, and 90 min after the administration for the healthy dog, and, e.g., 5 min, 40 min, 80 min, 120 min, and 180 min after the administration for other dogs.

The resultant first standard function is shown in Fig. 13.

Example 3: Formation of First Standard Function [CL × T - Cp/D Curve] for Human

Necessary data were read from graphs in the following three references, and a first standard function was formed following the same procedures as in Example 1.

(1) Fig. 2 in T.I. Ruo et al., Clinica Chimica Acta, Vol. 204, pp. 217 - 222 (1991): 1.95 g of inulin were intravenously administered once to one healthy human (human No. human 2) over 5 min, and the inulin concentrations in the plasma were measured at 18 time points up to 8 hours from the administration.

(2) Figs. 1 and 2 in H.F. Kuehnle et al., Nephron, Vol. 62, pp. 104 - 107 (1992): 5 g of inulin were administered into the vein of each of one healthy human (human No. human 3) and one patient suffering from a renal disease (human No. human 4), and the inulin concentrations in the serum samples were measured at nine time points up to four hours from the administration.

(3) Fig. 1 in K. Jung et al., Clin. Chem. Vol. 38, pp. 403 - 407 (1992): 5 g of inulin were administered to the vein of each of 21 patients (female: 12, male: 9, average age: 38) suffering from essential hypertension, diabetes mellitus, interstitial nephritis, and chronic glomerulonephritis. The inulin concentrations of the serum samples were measured eight times (human No. human 1) up to four hours after the administration.

The resultant first standard function is shown in Fig. 14.

Example 4: Measurement of Inulin Clearance for Rat (Method Using First Standard Function)

The first clearance calculation program 34 was executed. In this execution, the clearances of the fifteen rats in Example 1 were calculated by the first standard function (Fig. 12) obtained in Example 1, using the data for the respective blood collection times (except for the values obtained 5 min after the administration). More specifically, the ratio values of the inulin concentrations (Cp) in the plasma samples corresponding to the respective blood collection times to the inulin dose (D) were calculated, and the products of the clearance (CL) and the blood collection times (T) were calculated from the first standard function using the ratio values. The products were divided by the blood collection times (T), respectively, thereby obtaining the clearances (CL).

The calculation results are shown in Table 2 in comparison with the clearances obtained by the AUC method in Example 1. The ratio values of the clearances obtained by the method using the first standard function to the clearances obtained by the AUC method were averaged to be 1.04. The clearances obtained by the method using the first standard function were found almost equal to those obtained by the AUC method. Note that the standard deviation value of the ratio values was 15.1% of the average value.

[Table 2]

Comparison in Clearances of Rats between Conventional Method (AUC Method) and Method of Present Invention (Use of First Standard Function: Cp/D, CL × T)

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| N-1 | 15 | 1.71 | 2.37 | 1.39 |
| | 30 | | 2.42 | 1.42 |
| | 45 | | 2.15 | 1.26 |
| | 60 | | 1.86 | 1.09 |
| N-2 | 30 | 2.12 | 2.15 | 1.01 |
| | 60 | | 2.19 | 1.03 |
| | 90 | | 2.26 | 1.07 |
| | 120 | | 2.17 | 1.02 |
| N-3 | 30 | 2.71 | 2.82 | 1.04 |
| | 60 | | 2.73 | 1.01 |
| | 90 | | 2.87 | 1.06 |
| | 120 | | 2.83 | 1.04 |
| Uni-1 | 30 | 1.20 | 1.38 | 1.15 |
| | 60 | | 1.03 | 0.86 |
| | 90 | | 1.19 | 0.99 |
| | 120 | | 1.28 | 1.07 |
| Uni-2 | 30 | 1.47 | 1.37 | 0.93 |
| | 60 | | 1.83 | 1.24 |
| | 90 | | 1.77 | 1.20 |
| | 120 | | 1.70 | 1.16 |

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| CRF-1 | 30 | 0.63 | 0.62 | 0.98 |
| | 60 | | 0.57 | 0.90 |
| | 120 | | 0.63 | 1.00 |
| | 180 | | 0.65 | 1.03 |
| CRF-2 | 30 | 0.38 | 0.56 | 1.47 |
| | 60 | | 0.54 | 1.42 |
| | 120 | | 0.43 | 1.13 |
| | 180 | | 0.41 | 1.08 |
| CRF-3 | 30 | 0.44 | 0.50 | 1.14 |
| | 60 | | 0.59 | 1.34 |
| | 120 | | 0.46 | 1.05 |
| | 180 | | 0.48 | 1.09 |
| CRF-4 | 30 | 0.52 | 0.60 | 1.15 |
| | 60 | | 0.42 | 0.81 |
| | 120 | | 0.45 | 0.87 |
| | 180 | | 0.46 | 0.88 |
| CRF-5 | 30 | 1.05 | 0.95 | 0.90 |
| | 60 | | 1.01 | 0.96 |
| | 120 | | 1.00 | 0.95 |
| | 180 | | 1.09 | 1.04 |
| CRF-6 | 30 | 0.96 | 0.97 | 1.01 |
| | 60 | | 0.92 | 0.96 |
| | 120 | | 0.91 | 0.95 |
| | 180 | | 0.93 | 0.97 |
| CRF-7 | 30 | 1.25 | 1.13 | 0.90 |
| | 60 | | 1.19 | 0.95 |
| | 120 | | 1.27 | 1.02 |
| | 180 | | 1.25 | 1.00 |

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| CRF-8 | 30 | 1.16 | 1.17 | 1.01 |
| | 60 | | 1.09 | 0.94 |
| | 120 | | 1.06 | 0.91 |
| | 180 | | 1.10 | 0.95 |
| CRF-9 | 30 | 0.48 | 0.66 | 1.38 |
| | 60 | | 0.47 | 0.98 |
| | 120 | | 0.40 | 0.83 |
| | 180 | | 0.41 | 0.85 |
| CRF-10 | 30 | 0.58 | 0.63 | 1.09 |
| | 60 | | 0.47 | 0.81 |
| | 120 | | 0.46 | 0.79 |
| | 180 | | 0.61 | 1.05 |
| Average Value | | | | 1.04 |
| Standard Deviation | | | | 0.158 |
| Standard Deviation/Average Value | | | | 0.151 |

Example 5: Measurement of Inulin Clearance for Dog (Method Using First Standard Function)

The first clearance calculation program 34 was executed. In this execution, the clearances of the five dogs (two tests were conducted for dog Nos. 1919 and 1367 at an interval of two months) in Example 2 were calculated by the first standard function (Fig. 13) obtained in Example 2, using the data for the respective blood collection times (except for the values obtained 5 min after the administration). More specifically, the ratio values of the inulin concentrations (Cp) in the plasma samples corresponding to the respective blood collection times to the inulin dose (D) were calculated, and the products of the clearance (CL) and the blood collection times (T) were calculated from the first standard function using the ratio values. The products were divided by the blood collection times (T), respectively, thereby obtaining the clearances (CL).

The calculation results are shown in Table 3 in comparison with the clearances obtained by the AUC method in Example 2. The ratio values of the clearances obtained by the method using the first standard function to the clearances obtained by the AUC method were averaged to be 1.043. The clearances obtained by the method using the first standard function were found almost equal to those obtained by the AUC method. Note that the standard deviation value of the ratio values was 17.3% of the average value.

[Table 3]

Comparison in Clearances of Dogs between Conventional Method (AUC Method) and Method of Present Invention (Use of First Standard Function: Cp/D, CL × T)

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1919 | 20 | 52.4 | 48.6 | 0.93 |
| | 40 | | 58.9 | 1.12 |
| | 60 | | 61.7 | 1.18 |
| | 90 | | 57.3 | 1.09 |
| 1367 | 40 | 16.6 | 21.6 | 1.30 |
| | 80 | | 18.1 | 1.09 |
| | 120 | | 12.4 | 0.75 |
| | 180 | | 15.9 | 0.96 |
| 1919 | 20 | 57.2 | 52.4 | 0.92 |
| | 50 | | 63.5 | 1.11 |
| | 70 | | 63.7 | 1.11 |
| | 90 | | 56.1 | 0.98 |
| 1367 | 45 | 9.7 | 13.9 | 1.43 |
| | 80 | | 10.9 | 1.12 |
| | 115 | | 8.0 | 0.82 |
| | 180 | | 8.0 | 0.82 |
| 1253 | 45 | 22.2 | 21.9 | 0.99 |
| | 75 | | 27.3 | 1.23 |
| | 120 | | 27.3 | 1.23 |
| | 180 | | 25.8 | 1.16 |

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1104 | 40 | 10.9 | 15.5 | 1.42 |
| | 80 | | 10.3 | 0.94 |
| | 120 | | 9.7 | 0.89 |
| | 180 | | 8.5 | 0.78 |
| 963 | 20 | 36.6 | 33.7 | 0.92 |
| | 40 | | 31.1 | 0.85 |
| | 61 | | 36.3 | 0.99 |
| | 90 | | 36.8 | 1.01 |
| Average Value | | | | 1.043 |
| Standard Deviation | | | | 0.180 |
| Standard Deviation/Average Value | | | | 0.173 |

Example 6: Measurement of Inulin Clearance for Human (Method Using First Standard Function)

The first clearance calculation program 34 was executed. In this execution, the clearances of the four humans in Example 3 were calculated by the first standard function (Fig. 14) obtained in Example 3, using the data for the respective blood collection times. More specifically, the ratio values of the inulin concentrations (Cp) in the plasma samples corresponding to the respective blood collection times to the inulin dose (D) were calculated, and the products of the clearance (CL) and the blood collection times (T) were calculated from the first standard function using the ratio values. The products were divided by the blood collection times (T), respectively, thereby obtaining the clearances (CL).

The calculation results are shown in Table 4 in comparison with the clearances obtained by the AUC method in Example 3. The ratio values of the clearances obtained by the method using the first standard function to the clearances obtained by the AUC method were averaged to be 1.068. The clearances obtained by the method using the first standard function were found almost equal to those obtained by the AUC method. Note that the standard deviation value of the ratio values was 20.5% of the average value.

[Table 4]

Comparison in Clearances of Humans between Conventional Method (AUC Method) and Method of Present Invention (Use of First Standard Function: Cp/D, CL × T)

| Human No. | Blood Collection Time (min) | Conventional Method: Clearance (1) (mℓ/min) | Method of Present Invention: Clearance (2) (mℓ/min) | (2)/(1) (-) |
|---|---|---|---|---|
| Human 1 | 20 | 165 | 169 | 1.02 |
| | 30 | | 203 | 1.23 |
| | 45 | | 214 | 1.30 |
| | 90 | | 178 | 1.08 |
| | 120 | | 172 | 1.04 |
| | 150 | | 177 | 1.07 |
| | 180 | | 187 | 1.13 |
| | 240 | | 177 | 1.07 |
| Human 2 | 30 | 120 | 144 | 1.20 |
| | 45 | | 146 | 1.22 |
| | 60 | | 146 | 1.22 |
| | 90 | | 150 | 1.25 |
| | 120 | | 133 | 1.11 |
| | 150 | | 129 | 1.08 |
| | 180 | | 120 | 1.00 |
| | 240 | | 108 | 0.90 |
| | 300 | | 116 | 0.97 |
| | 360 | | 111 | 0.93 |
| | 420 | | 112 | 0.93 |
| | 480 | | 107 | 0.89 |

| Human No. | Blood Collection Time (min) | Conventional Method: Clearance (1) (mℓ/min) | Method of Present Invention: Clearance (2) (mℓ/min) | (2)/(1) (-) |
|---|---|---|---|---|
| Human 3 | 20 | 111 | 106 | 0.95 |
| | 33 | | 126 | 1.14 |
| | 40 | | 130 | 1.17 |
| | 120 | | 121 | 1.09 |
| | 150 | | 107 | 0.96 |
| | 180 | | 107 | 0.96 |
| | 210 | | 107 | 0.96 |
| | 240 | | 96.5 | 0.87 |
| Human 4 | 10 | 38.2 | 46.5 | 1.22 |
| | 20 | | 69.3 | 1.81 |
| | 33 | | 58.0 | 1.52 |
| | 40 | | 52.5 | 1.37 |
| | 120 | | 32.4 | 0.85 |
| | 150 | | 31.2 | 0.82 |
| | 180 | | 27.1 | 0.71 |
| | 210 | | 27.8 | 0.73 |
| | 240 | | 28.3 | 0.74 |
| Average Value | | | | 1.068 |
| Standard Deviation | | | | 0.219 |
| Standard Deviation/Average Value | | | | 0.205 |

Example 7: Formation of Second Standard Function [CL × T/Vss - Cp/(D/Vss) Curve] for Rat

The CL × T/Vss and Cp/(D/Vss) values corresponding to the blood collection times were calculated using the steady-state distribution volumes (Vss) (see Table 5 in Example 10) obtained from the Cp-T curve for the respective individuals used in Example 1, and the respective parameters of Example 1. These values were plotted to obtain the graph in Fig. 15. These points are apparently approximated by one curve from Fig. 15.

The second data accumulation program 35 and the second standard function formation program 36 were executed to form a second standard function.

Inulin clearances (CL) were calculated by the AUC method using a steady-state distribution volume (Vss) (see Table 5 in Example 10) obtained from a Cp-T curve and the respective parameters in Example 1 for the respective individuals used in Example 1. These data were applied to $Cp/(D/Vss) = A \times \exp(-\alpha \times (CL \times T)/Vss) + B \times \exp(-\beta \times (CL \times T)/Vss)$ to calculate the constants A, B, $\alpha$, and $\beta$, thereby forming a second standard function. This second standard function is shown in Fig. 15.

Example 8: Formation of Second Standard Function [CL × T/Vss - Cp/(D/Vss) Curve] for Dog

The CL × T/Vss and Cp/(D/Vss) values corresponding to the blood collection times were calculated using the steady-state distribution volumes (Vss) (see Table 7 in Example 11) obtained from the Cp-T curve for the respective individuals used in Example 2, and the respective parameters of Example 2. These values were plotted to obtain the graph in Fig. 16. These points are apparently approximated by one curve from Fig. 16.

The second data accumulation program 35 and the second standard function formation program 36 were executed to form a second standard function.

Inulin clearances (CL) were calculated by the AUC method using a steady-state distribution volume (Vss) (see Table 7 in Example 11) obtained from a Cp-T curve and the respective parameters in Example 2 for the respective individuals used in Example 2. These data were applied to $Cp/(D/Vss) = A \times \exp(-\alpha \times (CL \times T)/Vss) + B \times \exp(-\beta \times (CL \times T)/Vss)$ to calculate the constants A, B, $\alpha$ and $\beta$, thereby forming a second standard function. This second standard function is shown in Fig. 16.

Example 9: Formation of Second Standard Function [CL × T/Vss - Cp/(D/Vss) Curve] for Human

The CL × T/Vss and Cp/(D/Vss) values corresponding to the blood collection times were calculated using the steady-state distribution volumes (Vss) obtained from the Cp-T curve for the respective individuals used in Example 3, and the respective parameters of Example 3. These values were plotted to obtain the graph in Fig. 17. These points are apparently approximated by one curve from Fig. 17.

The second data accumulation program 35 and the second standard function formation program 36 were executed to form a second standard function.

Inulin clearances (CL) were calculated by the AUC method using a steady-state distribution volume (Vss) obtained from a Cp-T curve and the respective parameters in Example 3 for the respective individuals used in Example 3. These data were applied to $Cp/(D/Vss) = A \times \exp(-\alpha \times (CL \times T)/Vss) + B \times \exp(-\beta \times (CL \times T)/Vss)$ to calculate the constants A, B, $\alpha$ and $\beta$, thereby forming a second standard function. This second standard function is shown in Fig. 17.

Example 10: Measurement of Inulin Clearance for Rat (Method Using Second Standard Function)

The second clearance calculation program 37 was executed. In this execution, clearances were calculated by the second standard function (Fig. 15) obtained in Example 7, using the data for the blood collection times (except for the values obtained 5 min after the administration) of the fifteen rats in Example 1.

To estimate the Vss from the body weight (BW) of each rat, a conversion coefficient Ks = Vss/BW must be obtained. For this reason, in Example 1, the values Vss and BW of each rat obtained by the AUC method were used to obtain a ratio Vss/BW of each rat. The average value of the ratio values of the respective rats was defined as the conversion coefficient Ks. The results are shown in Table 5.

[Table 5]

| Rat. No. | BW (g) | Vss (mℓ) | Vss/BW |
|---|---|---|---|
| N-1 | 249 | 106.0 | 0.426 |
| N-2 | 250 | 60.9 | 0.244 |
| N-3 | 266 | 62.9 | 0.236 |
| Uni-1 | 271 | 68.5 | 0.253 |
| Uni-2 | 244 | 55.7 | 0.228 |
| CRF-1 | 251 | 54.8 | 0.218 |
| CRF-2 | 294 | 96.8 | 0.329 |
| CRF-3 | 306 | 81.0 | 0.265 |
| CRF-4 | 304 | 61.9 | 0.204 |
| CRF-5 | 246 | 64.1 | 0.261 |
| CRF-6 | 245 | 70.4 | 0.287 |
| CRF-7 | 294 | 66.2 | 0.225 |
| CRF-8 | 335 | 76.1 | 0.227 |
| CRF-9 | 328 | 74.5 | 0.227 |
| CRF-10 | 228 | 62.0 | 0.272 |
| | Average Value | | 0.260 |

[Table 5] (continued)

| Rat. No. | BW (g) | Vss (mℓ) | Vss/BW |
|---|---|---|---|
| | Standard Deviation | | 0.056 |

Assuming Ks = 0.260, the Vss of each rat was estimated by 0.260 × BW using the body weight BW. Cp/(D/Vss) was calculated using these values Vss, Cp, and D, and CL × T/Vss was obtained by the second standard function (Fig. 15) using the above calculation value. The value obtained by CL × T/Vss was divided by T/Vss to obtain a clearance (CL).

The calculation results are shown in Table 6 in comparison with the clearances obtained by the AUC method in Example 1. The ratio values of the clearances obtained by the method using the second standard function to the clearances obtained by the AUC method were averaged to be 1.026. The clearances obtained by the method using the second standard function were found almost equal to those obtained by the AUC method. Note that the standard deviation value of the ratio values was 14.0% of the average value.

[Table 6]

Comparison in Clearances of Rats between Conventional Method (AUC Method) and Method of Present Invention (Use of Second Standard Function: Cp/(D/Vss) CL × T/Vss)

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| N-1 | 15 | 1.71 | 2.40 | 1.40 |
| | 30 | | 2.52 | 1.47 |
| | 45 | | 2.16 | 1.26 |
| | 60 | | 1.84 | 1.08 |
| N-2 | 30 | 2.12 | 2.27 | 1.07 |
| | 60 | | 2.14 | 1.01 |
| | 90 | | 2.13 | 1.01 |
| | 120 | | 2.02 | 0.95 |
| N-3 | 30 | 2.71 | 2.92 | 1.08 |
| | 60 | | 2.71 | 1.00 |
| | 90 | | 2.80 | 1.03 |
| | 120 | | 2.72 | 1.00 |
| Uni-1 | 30 | 1.20 | 1.41 | 1.17 |
| | 60 | | 1.07 | 0.89 |
| | 90 | | 1.22 | 1.02 |
| | 120 | | 1.29 | 1.07 |
| Uni-2 | 30 | 1.47 | 1.47 | 1.00 |
| | 60 | | 1.80 | 1.22 |
| | 90 | | 1.68 | 1.14 |
| | 120 | | 1.57 | 1.07 |

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| CRF-1 | 30 | 0.63 | 0.61 | 0.97 |
| | 60 | | 0.59 | 0.94 |
| | 120 | | 0.66 | 1.04 |
| | 180 | | 0.64 | 1.01 |
| CRF-2 | 30 | 0.38 | 0.48 | 1.25 |
| | 60 | | 0.50 | 1.31 |
| | 120 | | 0.43 | 1.13 |
| | 180 | | 0.42 | 1.11 |
| CRF-3 | 30 | 0.44 | 0.40 | 0.91 |
| | 60 | | 0.54 | 1.23 |
| | 120 | | 0.46 | 1.05 |
| | 180 | | 0.50 | 1.14 |
| CRF-4 | 30 | 0.52 | 0.49 | 0.95 |
| | 60 | | 0.36 | 0.69 |
| | 120 | | 0.45 | 0.86 |
| | 180 | | 0.48 | 0.93 |
| CRF-5 | 30 | 1.05 | 0.98 | 0.93 |
| | 60 | | 1.07 | 1.02 |
| | 90 | | 1.01 | 0.96 |
| | 120 | | 1.06 | 1.01 |
| CRF-6 | 30 | 0.96 | 1.00 | 1.05 |
| | 60 | | 0.98 | 1.03 |
| | 120 | | 0.90 | 0.94 |
| | 180 | | 0.88 | 0.92 |
| CRF-7 | 30 | 1.25 | 1.06 | 0.85 |
| | 60 | | 1.24 | 0.99 |
| | 120 | | 1.32 | 1.06 |
| | 180 | | 1.30 | 1.04 |

| Rat No. | Blood Collection Time (min) | Conventional Method: Clearance (1) (mℓ/min) | Method of Present Invention: Clearance (2) (mℓ/min) | (2)/(1) (-) |
|---|---|---|---|---|
| CRF-8 | 30 | 1.16 | 1.00 | 0.86 |
| | 60 | | 1.09 | 0.94 |
| | 120 | | 1.16 | 1.00 |
| | 180 | | 1.24 | 1.07 |
| CRF-9 | 30 | 0.48 | 0.51 | 1.06 |
| | 60 | | 0.39 | 0.80 |
| | 120 | | 0.38 | 0.79 |
| | 180 | | 0.42 | 0.87 |
| CRF-10 | 30 | 0.58 | 0.67 | 1.15 |
| | 60 | | 0.50 | 0.85 |
| | 120 | | 0.49 | 0.84 |
| | 180 | | 0.59 | 1.01 |
| Average Value | | | | 1.026 |
| Standard Deviation | | | | 0.144 |
| Standard Deviation/Average Value | | | | 0.140 |

Example 11: Measurement of Inulin Clearance for Dog (Method Using Second Standard Function)

The second clearance calculation program 37 was executed. In this execution, clearances were calculated by the second standard function (Fig. 16) obtained in Example 8, using the data for the blood collection times (except for the values obtained 5 min after the administration) of the five dogs in Example 2.

To estimate the Vss from the body weight (BW) of each dog, a conversion coefficient Ks = Vss/BW must be obtained. For this reason, in Example 2, the values Vss and BW of each dog obtained by the AUC method were used to obtain a ratio Vss/BW of each dog. The average value of the ratio values of the respective dogs was defined as the conversion coefficient Ks. The results are shown in Table 7.

[Table 7]

| Dog. No. | BW (g) | Vss (mℓ) | Vss/BW |
|---|---|---|---|
| 1367 | 10000 | 2510 | 0.251 |
| 1919 | 12500 | 1545 | 0.124 |
| 1367 | 8500 | 2185 | 0.257 |
| 1919 | 13000 | 1950 | 0.150 |
| 1253 | 10000 | 1544 | 0.154 |
| 1104 | 14000 | 1934 | 0.138 |
| 963 | 10000 | 1544 | 0.154 |
| | Average Value | | 0.176 |
| | Standard Deviation | | 0.051 |

Assuming Ks = 0.176, the Vss of each dog was estimated by 0.176 × BW using the body weight BW. Cp/(D/Vss) was calculated using these values Vss, Cp, and D, and CL × T/Vss was obtained by the second standard function (Fig. 16) using the above calculation value. The value obtained by CL × T/Vss was divided by T/Vss to obtain a clearance (CL).

The calculation results are shown in Table 8 in comparison with the clearances obtained by the AUC method in Example 2. The ratio values of the clearances obtained by the method using the second standard function to the clearances obtained by the AUC method were averaged to be 1.065. The clearances obtained by the method using the second standard function were found almost equal to those obtained by the AUC method. Note that the standard deviation value of the ratio values was 17.2% of the average value.

[Table 8]

Comparison in Clearances of Dogs between Conventional Method (AUC Method) and Method of Present Invention (Use of Second Standard Function: Cp/(D/Vss) CL × T/Vss)

| Dog No. | Blood Collection Time (min) | Conven-tional Method: Clearance (1) (mℓ/min) | Method of Present Invention: Clearance (2) (mℓ/min) | (2)/(1) (-) |
|---|---|---|---|---|
| 1919 | 20 | 52.4 | 46.9 | 0.90 |
| | 40 | | 62.3 | 1.19 |
| | 60 | | 64.3 | 1.23 |
| | 90 | | 59.2 | 1.13 |
| 1367 | 40 | 16.6 | 22.5 | 1.35 |
| | 80 | | 20.2 | 1.22 |
| | 120 | | 13.7 | 0.82 |
| | 180 | | 15.8 | 0.95 |
| 1919 | 20 | 57.2 | 50.4 | 0.88 |
| | 50 | | 67.1 | 1.17 |
| | 70 | | 67.3 | 1.18 |
| | 90 | | 59.3 | 1.04 |
| 1367 | 45 | 9.7 | 14.5 | 1.49 |
| | 80 | | 12.2 | 1.26 |
| | 115 | | 9.1 | 0.94 |
| | 180 | | 9.1 | 0.94 |
| 1253 | 45 | 22.2 | 23.5 | 1.06 |
| | 75 | | 29.0 | 1.30 |
| | 120 | | 26.5 | 1.19 |
| | 180 | | 23.9 | 1.08 |

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1104 | 40 | 10.9 | 13.3 | 1.22 |
| | 80 | | 9.3 | 0.86 |
| | 120 | | 9.1 | 0.83 |
| | 180 | | 8.1 | 0.74 |
| 963 | 20 | 36.6 | 33.3 | 0.91 |
| | 40 | | 34.6 | 0.94 |
| | 61 | | 37.9 | 1.04 |
| | 90 | | 35.6 | 0.97 |
| Average Value | | | | 1.065 |
| Standard Deviation | | | | 0.183 |
| Standard Deviation/Average Value | | | | 0.172 |

Since the values of the body weights of the humans were unknown according to the references, no clearances were calculated from the second standard function (Fig. 17) obtained in Example 9.

Example 12: Formation of First Standard Function for Dog in Use of Meglumine Iothalamate as Test Substance

The first data accumulation program 32 and the first standard function formation program 33 were executed to form a first standard function.

A total of five female beagle dogs (body weight: 8.5 to 14 kg) consisting of one healthy dog (dog No. 1919), three partially nephrectomized dogs (chronic renal failure model) (dog Nos. 1367, 1253, and 1104) were used.

A 3% inulin-containing iothalamate solution was administered once in the amount of 0.3 mℓ/kg to each beagle dog from its foreleg vein. The meglumine iothalamate in the 3% inulin-containing iothalamate solution was obtained by diluting a conray injection solution (60 W/V%) (meglumine iothalamate injection solution of the Japanese Pharmacopoeia, available from DAIICHI PHARMACEUTICAL CO., LTD.) with saline to adjust the concentration to 3%.

Blood collection operations were performed five times with intervals after the administration. The blood collection times were, e.g., 5 min, 20 min, 40 min, 60 min, and 90 min after the administration for the healthy dog, and, e.g., 5 min, 40 min, 80 min, 120 min, and 180 min after the administration for other dogs.

The concentration of meglumine iothalamate in the plasma was measured as follows. 400 μℓ of a 0.1N-HCl-methanol (1 : 9) solution mixture were added to 100 μℓ of a dog plasma sample, and the resultant solution was stirred well and subjected to centrifugal separation at 16,000 rpm for 10 min. The resultant supernatant was analyzed by liquid chromatography. The column of the liquid chromatography was a TOSO-ODS-80TM (4.6 × 150 mm, 40°C), and the mobile phase was a solution obtained by dissolving phosphoric acid (5 mℓ) and ammonium formate (1.0 g) in a 4% aqueous acetonitrile solution (1 ℓ). Detection was performed by 235-nm ultraviolet absorption.

A first standard function [CL × T - Cp/D curve] was formed following the same procedures as in Example 1. The result is shown in Fig. 18.

Example 13: Measurement of Meglumine Iothalamate Clearance for Dog (Method Using First Standard Function)

The first clearance calculation program 34 was executed. In this execution, the clearances of the four dogs (two tests were conducted for dog Nos. 1919 and 1367 at an interval of two months) in Example 12 were calculated by the first standard function (Fig. 18) obtained in Example 12, using the data for the respective blood collection times (except for the values obtained 5 min after the administration). More specifically, the ratio values of the meglumine iothalamate concentrations (Cp) in the plasma samples corresponding to the respective blood collection times to the meglumine iothalamate dose (D) were calculated, and the products of the clearance (CL) and the blood collection times (T) were calculated from the first standard function using the ratio values. The products were divided by the blood collection times (T), respectively, thereby obtaining the clearances (CL).

The calculation results are shown in Table 9 in comparison with the clearances obtained by the AUC method in Example 12. The ratio values of the clearances obtained by the method using the first standard function to the clearances obtained by the AUC method were averaged to be 1.044. The clearances obtained by the method using the first standard function were found almost equal to those obtained by the AUC method. Note that the standard deviation value of the ratio values was 21.6% of the average value.

[Table 9]

Comparison for Iothalamic Acid in Clearances of Dogs between Conventional Method (AUC Method) and Method of Present Invention (Use of First Standard Function: Cp/D, CL × T)

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1919 | 20 | 49.1 | 57.4 | 1.17 |
| | 40 | | 53.0 | 1.08 |
| | 60 | | 52.5 | 1.07 |
| | 90 | | 43.9 | 0.89 |
| 1367 | 40 | 21.6 | 25.5 | 1.18 |
| | 80 | | 21.1 | 0.98 |
| | 120 | | 19.4 | 0.90 |
| | 180 | | 19.5 | 0.90 |
| 1919 | 20 | 45.6 | 52.1 | 1.14 |
| | 50 | | 48.9 | 1.07 |
| | 70 | | 48.4 | 1.06 |
| | 90 | | 42.0 | 0.92 |
| 1367 | 45 | 7.0 | 12.2 | 1.73 |
| | 80 | | 6.9 | 0.98 |
| | 115 | | 8.5 | 1.22 |
| | 180 | | 6.4 | 0.92 |
| 1253 | 45 | 20.5 | 20.7 | 1.01 |
| | 75 | | 23.6 | 1.15 |
| | 120 | | 24.4 | 1.19 |
| | 180 | | 22.7 | 1.11 |

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1104 | 40 | 11.8 | 12.7 | 1.08 |
| | 80 | | 4.7 | 0.39 |
| | 120 | | 12.6 | 1.07 |
| | 180 | | 9.9 | 0.84 |
| Average Value | | | | 1.044 |
| Standard Deviation | | | | 0.225 |
| Standard Deviation/Average Value | | | | 0.216 |

Example 14: Comparison between Conventional Method (Creatinine Clearance) and [Method Using First or Second Standard Function] for Rat

The first or second clearance calculation program 34 or 37 was executed using the first standard function (Fig. 12) obtained in Example 1 or the second standard function (Fig. 15) obtained in Example 7. Inulin clearance measurements of rats different from those used for the function formation in Example 1 or 7 were performed, and the results were compared with the creatinine clearance values obtained by the conventional method.

The rats (female SD rats) were seven partially nephrectomized rats. The inulin was administered following the same procedures as in Example 1. 0.2 mℓ of blood was collected from each sample 113 or 114 min after the administration, and the plasma was immediately separated from the serum in each sample. The inulin concentration of each plasma sample was measured following the same procedures as in Example 1. The inulin clearances were calculated using each function as in Example 4 or 10.

Each creatinine clearance was measured by the conventional method as follows.

0.25 mℓ of blood was collected from the jugular vein of each rat under ether anesthesia and sampled in a heparin-containing Spitz tube, and the plasma was immediately separated from the blood. On the other hand, urine was accumulated using a urinary metabolism cage, and the amount of urine was accurately measured. The creatinine concentrations in the plasma and urea were measured by Creatinine Analyzer 2 (BECKMAN). The creatinine clearance (CCr) was calculated by the following formula using the resultant data.

CCr = Amount of Urine for 24 Hours (mℓ/24 × 60 min) × Creatinine Concentration in Urea/Creatinine

Concentration in Plasma

The calculation results are shown in Tables 10 and 11. When the first or second standard function was used, clearance values equal to those in the conventional method could be obtained by single administration and single blood collection without performing urine collection performed in the conventional method.

[Table 10]

| Comparison in Clearances of Rats between Conventional Method (24-Hour Urine Accumulation Method) and Method of Present Invention (Use of First Standard Function: Cp/D, CL × T) | | | | |
|---|---|---|---|---|
| Rat. No. | Conventional Method: (24-Hour Urine Accumulation Method) Creatinine Clearance (1) (ml/min) | Time from Administration to Blood Collection (min) | Method of present Invention: Inulin Clearance (2) (mℓ/min) | (2) / (1) (-) |
| R32 | 0.44 | 114 | 0.40 | 0.913 |

[Table 10] (continued)

| Comparison in Clearances of Rats between Conventional Method (24-Hour Urine Accumulation Method) and Method of Present Invention (Use of First Standard Function: Cp/D, CL × T) | | | | |
|---|---|---|---|---|
| Rat. No. | Conventional Method: (24-Hour Urine Accumulation Method) Creatinine Clearance (1) (ml/min) | Time from Administration to Blood Collection (min) | Method of present Invention: Inulin Clearance (2) (mℓ/min) | (2) / (1) (-) |
| R35 | 0.71 | 114 | 0.67 | 0.949 |
| B47 | 0.70 | 114 | 0.63 | 0.901 |
| R102 | 0.32 | 114 | 0.27 | 0.839 |
| B3 | 0.65 | 113 | 0.70 | 1.074 |
| R93 | 0.44 | 113 | 0.55 | 1.247 |
| R66 | 0.88 | 113 | 0.91 | 1.034 |
| | | | Average Value | 0.994 |
| | | | Standard Deviation | 0.137 |
| | | | Standard Deviation/Average Value | 0.138 |

[Table 11]

| Comparison in Clearances of Rats between Conventional Method (24-Hour Urine Accumulation Method) and Method of Present Invention (Use of Second Standard Function: Cp/(D/Vss), CL × T/Vss) | | | | |
|---|---|---|---|---|
| Rat. No. | Conventional Method: (24-Hour Urine Accumulation Method) Creatinine Clearance (1) (ml/min) | Time from Administration to Blood Collection (min) | Method of present Invention: Inulin Clearance (2) (mℓ/min) | (2) / (1) (-) |
| R32 | 0.44 | 114 | 0.39 | 0.890 |
| R35 | 0.71 | 114 | 0.70 | 0.992 |
| B47 | 0.70 | 114 | 0.65 | 0.930 |
| R102 | 0.32 | 114 | 0.26 | 0.807 |
| B3 | 0.65 | 113 | 0.73 | 1.120 |
| R93 | 0.44 | 113 | 0.56 | 1.270 |
| R66 | 0.88 | 113 | 0.95 | 1.080 |
| | | | Average Value | 1.013 |
| | | | Standard Deviation | 0.156 |
| | | | Standard Deviation/Average Value | 0.154 |

Example 15: Comparison between AUC Method and [Method Using First or Second Standard Function] for Dog

The first or second clearance calculation program 34 or 37 was executed using the first standard function (Fig. 13) obtained in Example 2 or the second standard function (Fig. 16) obtained in Example 8. Inulin clearance measurements of dogs different from those used for the function formation in Example 2 or 8 were performed, or even if the same dogs were used, the test timings were different from those of formation of the above standard functions. The results were compared with the creatinine clearance values obtained by the AUC method.

A total of six female beagle dogs consisting of one healthy dog (dog No. 1919) and five unilaterally nephrectomized dogs were used in this measurement. Inulin administration and blood collection were performed following the same procedures as in Example 2. The inulin concentration in each plasma sample was measured following the same procedures as in Example 1. The inulin clearances were calculated from the respective functions following the same procedures as in Examples 5 and 11.

The inulin clearances were measured by the AUC method following the same procedures as in Example 1.

The calculation results are shown in Tables 12 and 13. When the first or second standard function was used, clearance values equal to those of the AUC method could be obtained by single administration and single blood collection without frequent blood collection required in the AUC method.

[Table 12]

Comparison in Clearances of Dogs between Conventional Method (AUC Method) and Method of Present Invention (Use of First Standard Function: Cp/D, CL × T)

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1919 | 20 | 52.0 | 62.5 | 1.202 |
| | 40 | | 65.1 | 1.252 |
| | 60 | | 58.1 | 1.117 |
| | 90 | | 50.6 | 0.973 |
| 963 | 20 | 40.8 | 42.1 | 1.032 |
| | 40 | | 34.8 | 0.853 |
| | 60 | | 35.6 | 0.873 |
| | 90 | | 33.7 | 0.826 |
| 1101 | 15 | 45.3 | 42.9 | 0.947 |
| | 30 | | 34.6 | 0.763 |
| | 60 | | 38.0 | 0.839 |
| | 120 | | 40.3 | 0.889 |
| | 180 | | 44.1 | 0.974 |
| 1102 | 15 | 33.2 | 32.6 | 0.981 |
| | 30 | | 28.1 | 0.846 |
| | 60 | | 27.7 | 0.834 |
| | 120 | | 33.2 | 0.999 |
| | 180 | | 33.5 | 1.010 |
| 1103 | 15 | 32.5 | 39.5 | 1.214 |
| | 60 | | 26.6 | 0.819 |
| | 120 | | 28.0 | 0.862 |
| | 180 | | 27.8 | 0.854 |

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1105 | 15 | 31.2 | 33.3 | 1.067 |
| | 30 | | 28.8 | 0.922 |
| | 60 | | 26.8 | 0.859 |
| | 120 | | 30.6 | 0.979 |
| | 180 | | 27.4 | 0.879 |
| Average Value | | | | 0.951 |
| Standard Deviation | | | | 0.129 |
| Standard Deviation/Average Value | | | | 0.136 |

[Table 13]

Comparison in Clearances of Dogs between Conventional Method (AUC Method) and Method of Present Invention (Use of Second Standard Function: Cp/(D/Vss), CL × T/Vss)

| Dog No. | Blood Collection Time (min) | Conven-tional Method: Clearance (1) (mℓ/min) | Method of Present Invention: Clearance (2) (mℓ/min) | (2)/(1) (-) |
|---|---|---|---|---|
| 1919 | 20 | 52.0 | 62.2 | 1.196 |
| | 40 | | 68.9 | 1.325 |
| | 60 | | 61.3 | 1.179 |
| | 90 | | 53.6 | 1.031 |
| 963 | 20 | 40.8 | 43.5 | 1.066 |
| | 40 | | 38.7 | 0.949 |
| | 60 | | 37.5 | 0.919 |
| | 90 | | 33.3 | 0.816 |
| 1101 | 15 | 45.3 | 39.6 | 0.874 |
| | 30 | | 34.9 | 0.769 |
| | 60 | | 40.2 | 0.887 |
| | 120 | | 40.2 | 0.887 |
| | 180 | | 43.1 | 0.950 |
| 1102 | 15 | 33.2 | 30.6 | 0.923 |
| | 30 | | 28.6 | 0.863 |
| | 60 | | 30.3 | 0.912 |
| | 120 | | 31.7 | 0.955 |
| | 180 | | 30.8 | 0.928 |

| Dog No. | Blood Collection Time (min) | Conventional Method: Clearance (1) (mℓ/min) | Method of Present Invention: Clearance (2) (mℓ/min) | (2)/(1) (-) |
|---|---|---|---|---|
| 1103 | 15 | 32.5 | 37.1 | 1.140 |
| | 60 | | 29.1 | 0.895 |
| | 120 | | 27.9 | 0.860 |
| | 180 | | 26.6 | 0.819 |
| 1105 | 15 | 31.2 | 29.9 | 0.957 |
| | 30 | | 27.8 | 0.891 |
| | 60 | | 28.6 | 0.917 |
| | 120 | | 31.1 | 0.996 |
| | 180 | | 27.4 | 0.878 |
| Average Value | | | | 0.955 |
| Standard Deviation | | | | 0.128 |
| Standard Deviation/Average Value | | | | 0.134 |

Note that $Cp/D = A \times \exp(-\alpha \times (CL \times T)) + B \times \exp(-\beta \times (CL \times T))$ is an example of the first standard function $Cp/D = f_1(CL \times T)$. Similarly, $Cp/(D/Vss) = A \times \exp(-\alpha \times (CL \times T)/Vss) + B \times \exp(-\beta \times (CL \times T)/Vss)$ is an example of the second standard function $Cp/(D/Vss) = f_2((CL \times T)/Vss)$.

As has been described above, according to the clearance measuring apparatus and method of the present invention, a clearance can be accurately measured by single intravenous injection and single blood collection without requiring urine collection while the blood collection time is arbitrarily selected. In addition, measurement of an inulin clearance is combined with a method of highly sensitively measuring the inulin concentration in plasma to reduce the inulin dose. Therefore, the pains and load on a patient and the load on a physician can be greatly reduced.

Further, according to a computer program product (clearance measurement storage medium) according to the present invention, a clearance measurement program stored in a program area is executed to highly accurately measure the clearance by single phlebocylsys and single blood collection. Measurement of an inulin clearance can be combined with a method of highly sensitively measuring the inulin concentration in plasma to allow a reduction in inulin dose. In this manner, any pain and the load of the patient and the load of the physician can be greatly reduced.

Furthermore, when the first or second standard curve of the present invention is used, clearances can be highly accurately measured by single intravenous injection and single blood collection while arbitrarily selecting the blood collection time without requiring urine collection. In addition, inulin clearance measurement can be combined with a method of highly sensitively measuring an inulin concentration in plasma, thereby reducing the inulin dose. In this manner, the pains and load of a patient and the load of a physician can be greatly reduced.

From the invention thus described, it will be obvious that the invention may be varied in many ways. Such variations are not to be regarded as a departure from the spirit and scope of the invention, and all such modifications as would be obvious to one skilled in the art are intended to be included within the scope of the following claims.

The basic Japanese Applications No.108306/1995 filed on April 7, 1995, No.230365/1995 filed on September 7, 1995, No.230387/1995 filed on September 7, 1995 and No.289961/1995 filed on November 8, 1995 are hereby incorporated by reference.

## Claims

1. A clearance measuring apparatus for measuring a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said apparatus comprising:

   storage means for storing a first standard function which takes, as one parameter, a product value of the clearance (CL) and the blood collection time (T) and, as the other parameter, a value obtained by dividing the test substance concentration (Cp) by the dose (D);
   input means for receiving inputs of the dose (D), the blood collection time (T), and the test substance concentration (Cp); and
   clearance calculation means for calculating the clearance (CL) by applying the dose (D), the blood collection time (T) and the test substance concentration (Cp) received by said input means to the first standard function stored in said storage means.

2. A clearance measuring apparatus according to claim 1, wherein the first standard function is expressed by the following equation:

$$Cp/D = A \times \exp(-\alpha \times (CL \times T))$$

$$+ B \times \exp(-\beta \times (CL \times T))$$

   (where A, B, $\alpha$ and $\beta$ are constants specified for each animal species).

3. A clearance measuring apparatus for measuring a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said apparatus comprising:

   storage means for storing a second standard function which takes, as one parameter, a value obtained by dividing a product value of the clearance (CL) and the blood collection time (T) by the steady-state distribution volume (Vss) and, as the other parameter, a value obtained by dividing the test substance concentration (Cp) by a value obtained by dividing the dose (D) by the steady-state distribution volume (Vss);
   input means for receiving inputs of the dose (D), the blood collection time (T), the test substance concentration (Cp) and the steady-state distribution volume (Vss); and
   clearance calculation means for calculating the clearance (CL) by applying the dose (D), the blood collection time (T), the test substance concentration (Cp) and the steady-state distribution volume (Vss) received by said input means to the second standard function stored in said storage means.

4. A clearance measuring apparatus according to claim 3, wherein the second standard function is expressed by the following equation:

$$Cp/(D/Vss) = A \times \exp(-\alpha \times (CL \times T)/Vss)$$

$$+ B \times \exp(-\beta \times (CL \times T)/Vss)$$

   (where A, B, $\alpha$ and $\beta$ are constants specified for each animal species).

5. A clearance measuring method for measuring a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said method comprising the step of:
   calculating the clearance (CL) by applying the dose (D), the blood collection time (T) and the test substance concentration (Cp) to a first standard function which takes, as one parameter, a product value of the clearance (CL) and the blood collection time (T) and, as the other parameter, a value obtained by dividing the test substance concentration (Cp) by the dose (D).

**6.** A clearance measuring method according to claim 5, wherein the first standard function is expressed by the following equation:

$$Cp/D = A \times \exp(-\alpha \times (CL \times T))$$
$$+ B \times \exp(-\beta \times (CL \times T))$$

(where A, B, $\alpha$ and $\beta$ are constants specified for each animal species).

**7.** A clearance measuring method for measuring a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said method comprising the step of:

calculating the clearance (CL) by applying the dose (D), the blood collection time (T), the test substance concentration (Cp) and the steady-state distribution volume (Vss) to a second standard function which takes, as one parameter, a value obtained by dividing a product value of the clearance (CL) and the blood collection time (T) by the steady-state distribution volume (Vss) and, as the other parameter, a value obtained by dividing the test substance concentration (Cp) by a value obtained by dividing the dose (D) by the steady-state distribution volume (Vss).

**8.** A clearance measuring method according to claim 7, wherein the second standard function is expressed by the following equation:

$$Cp/(D/Vss) = A \times \exp(-\alpha \times (CL \times T)/Vss)$$
$$+ B \times \exp(-\beta \times (CL \times T)/Vss)$$

(where A, B, $\alpha$ and $\beta$ are constants specified for each animal species).

**9.** A computer program product for use with an information processing apparatus, said computer program product comprising:

a computer usable medium having a program area and a computer readable program embodied in said program area of said medium for measuring a clearance, said computer program product having:

a computer readable input routine for causing said computer to receive inputs of a dose (D) of a test substance administered to a test subject, a blood collection time (T) required from administration of the test substance to blood collection with respect to the test subject, and a test substance concentration (Cp) in plasma which is obtained from blood collected from the test subject, and

a computer readable arithmetic routine for causing said computer to apply the dose (D), the blood collection time (T) and the test substance concentration (Cp) input in the input routine to a first standard function which takes, as one parameter, a product value of a clearance (CL) of the test substance and the blood collection time (T) and, as the other parameter, a value obtained by dividing the test substance concentration (Cp) by the dose (D), thereby calculating the clearance (CL).

**10.** A computer program product according to claim 9, wherein the first standard function is expressed by the following equation:

$$Cp/D = A \times \exp(-\alpha \times (CL \times T))$$
$$+ B \times \exp(-\beta \times (CL \times T))$$

(where A, B, $\alpha$ and $\beta$ are constants specified for each animal species).

**11.** A computer program product for use with an information processing apparatus, said computer program product comprising:

a computer usable medium having a program area and a computer readable program embodied in said program area of said medium for measuring a clearance, said computer program product having:
a computer readable input routine for causing said computer to receive inputs of a dose (D) of a test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, and
a computer readable arithmetic routine for causing said computer to apply the dose (D), the blood collection time (T), the test substance concentration (Cp) and the steady-state distribution volume (Vss) input in the input routine to a second standard function which takes, as one parameter, a value obtained by dividing a product value of a clearance (CL) of the test substance and the blood collection time (T) by the steady-state distribution volume (Vss) and, as the other parameter, a value obtained by dividing the test substance concentration (Cp) by a value obtained by dividing the dose (D) by the steady-state distribution volume (Vss), thereby calculating the clearance (CL).

**12.** A computer program product according to claim 11, wherein the second standard function is expressed by the following equation:

$$Cp/(D/Vss) = A \times \exp(-\alpha \times (CL \times T)/Vss) + B \times \exp(-\beta \times (CL \times T)/Vss)$$

(where A, B, $\alpha$ and $\beta$ are constants specified for each animal species).

**13.** A clearance measurement standard curve for measuring a clearance (CL) of a test substance on the basis of a dose (D) of the test substance administered to a test subject, a blood collection time (T) required from administration of the test substance to blood collection with respect to the test subject, and a test substance concentration (Cp) in plasma which is obtained from blood collected from the test subject, said curve characterized in that a product value of the time (T) required from administration of a test substance to blood collection and the clearance (CL) is plotted along one of coordinate axes, and a ratio value of the test substance concentration (Cp) in plasma to the test substance dose (D) is plotted along the other of the coordinate axes.

**14.** A clearance measurement standard curve for measuring a clearance (CL) of a test substance on the basis of a dose (D) of the test substance administered to a test subject, a blood collection time (T) required from administration of the test substance to blood collection with respect to the test subject, a test substance concentration (Cp) in plasma which is obtained from blood collected from the test subject, and a steady-state distribution volume (Vss) of the test subject, said curve characterized in that a value obtained by dividing a product value of the time (T) required from administration of a test substance to blood collection and the clearance (CL) by the steady-state distribution volume (Vss) is plotted along one of coordinate axes, and a value obtained by dividing the test substance concentration (Cp) in plasma by a ratio value of the test substance dose (D) to the steady-state distribution volume (Vss).

Fig.1

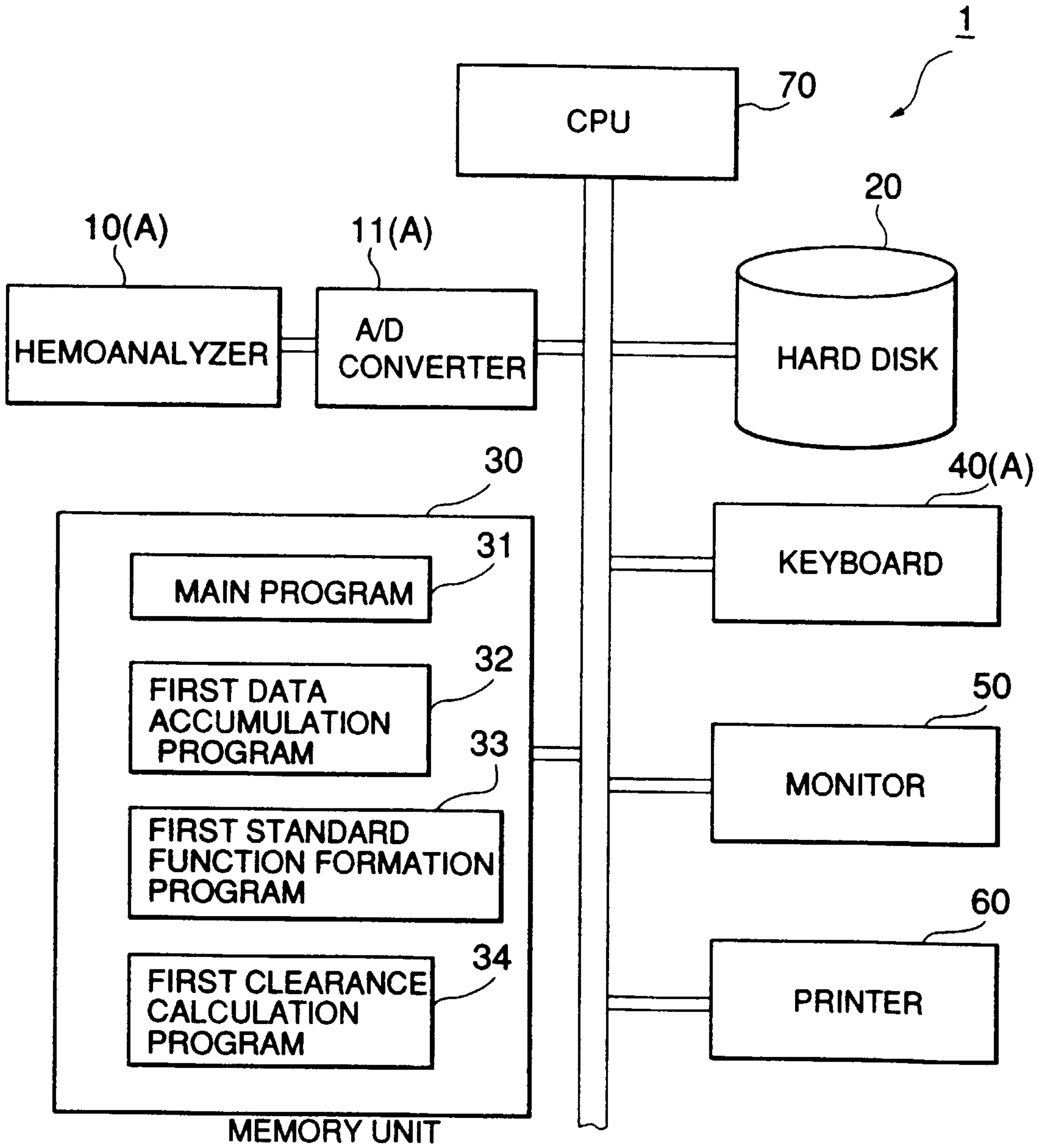
1
CPU
70
20
10(A)
11(A)
HEMOANALYZER
A/D
CONVERTER
HARD DISK
30
40(A)
31
MAIN PROGRAM
KEYBOARD
32
FIRST DATA
ACCUMULATION
PROGRAM
50
33
MONITOR
FIRST STANDARD
FUNCTION FORMATION
PROGRAM
60
34
FIRST CLEARANCE
CALCULATION
PROGRAM
PRINTER
MEMORY UNIT

# Fig.2

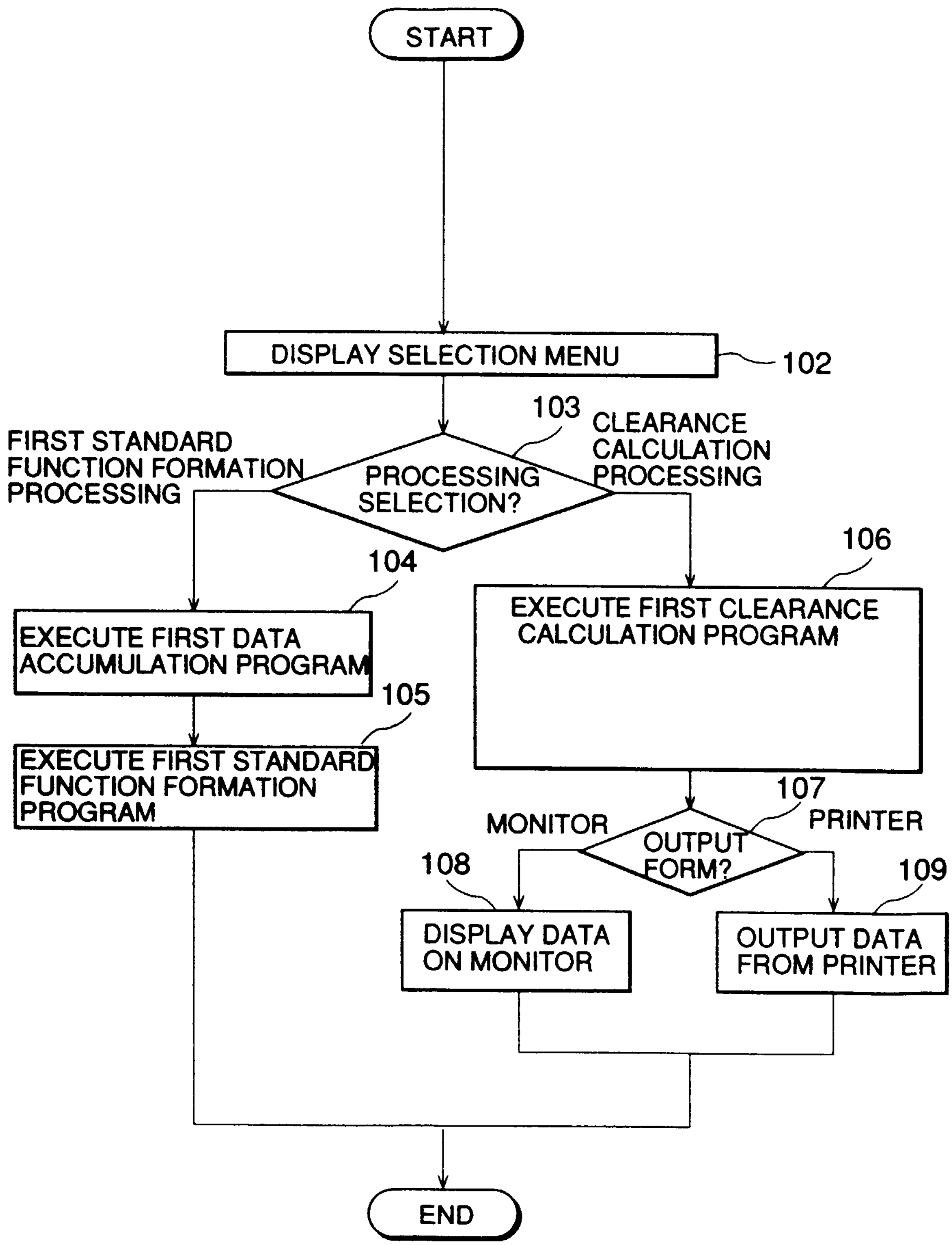
START
DISPLAY SELECTION MENU
102
103
FIRST STANDARD
FUNCTION FORMATION
PROCESSING
PROCESSING
SELECTION?
CLEARANCE
CALCULATION
PROCESSING
104
EXECUTE FIRST DATA
ACCUMULATION PROGRAM
105
EXECUTE FIRST STANDARD
FUNCTION FORMATION
PROGRAM
106
EXECUTE FIRST CLEARANCE
CALCULATION PROGRAM
107
MONITOR
OUTPUT
FORM?
PRINTER
108
DISPLAY DATA
ON MONITOR
109
OUTPUT DATA
FROM PRINTER
END

Fig.3

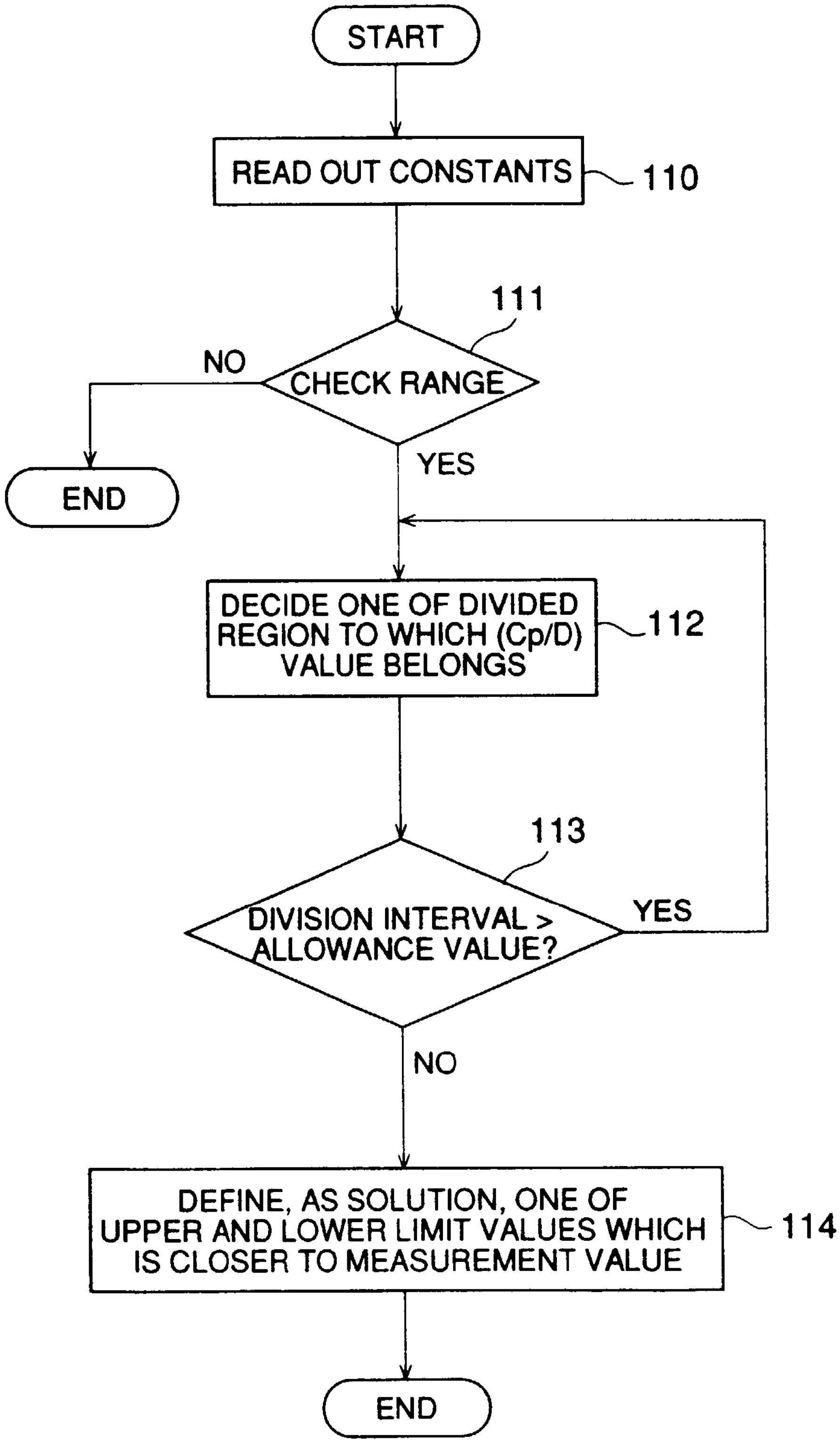
START
READ OUT CONSTANTS
110
111
CHECK RANGE
NO
END
YES
DECIDE ONE OF DIVIDED REGION TO WHICH (Cp/D) VALUE BELONGS
112
113
DIVISION INTERVAL > ALLOWANCE VALUE?
YES
NO
DEFINE, AS SOLUTION, ONE OF UPPER AND LOWER LIMIT VALUES WHICH IS CLOSER TO MEASUREMENT VALUE
114
END

Fig.4

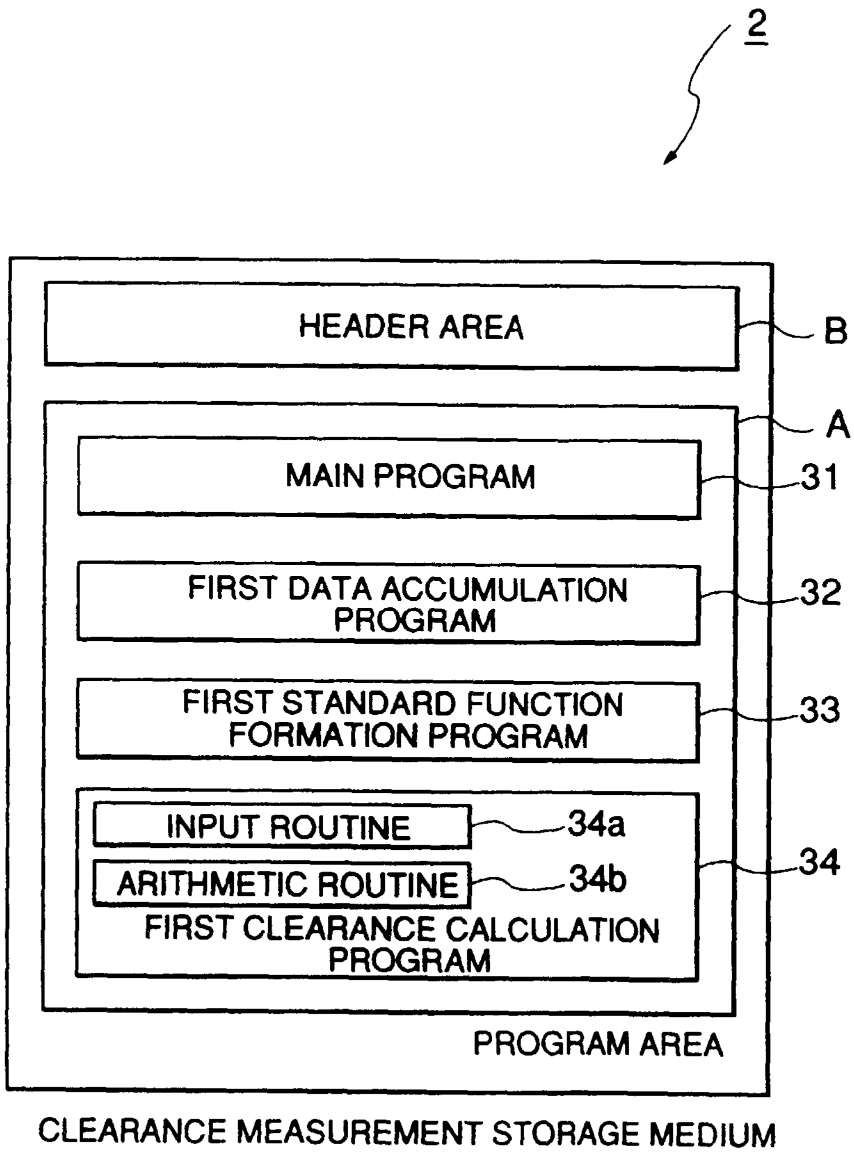
2
HEADER AREA
B
A
MAIN PROGRAM
31
FIRST DATA ACCUMULATION PROGRAM
32
FIRST STANDARD FUNCTION FORMATION PROGRAM
33
INPUT ROUTINE
34a
ARITHMETIC ROUTINE
34b
FIRST CLEARANCE CALCULATION PROGRAM
34
PROGRAM AREA
CLEARANCE MEASUREMENT STORAGE MEDIUM

Fig.5

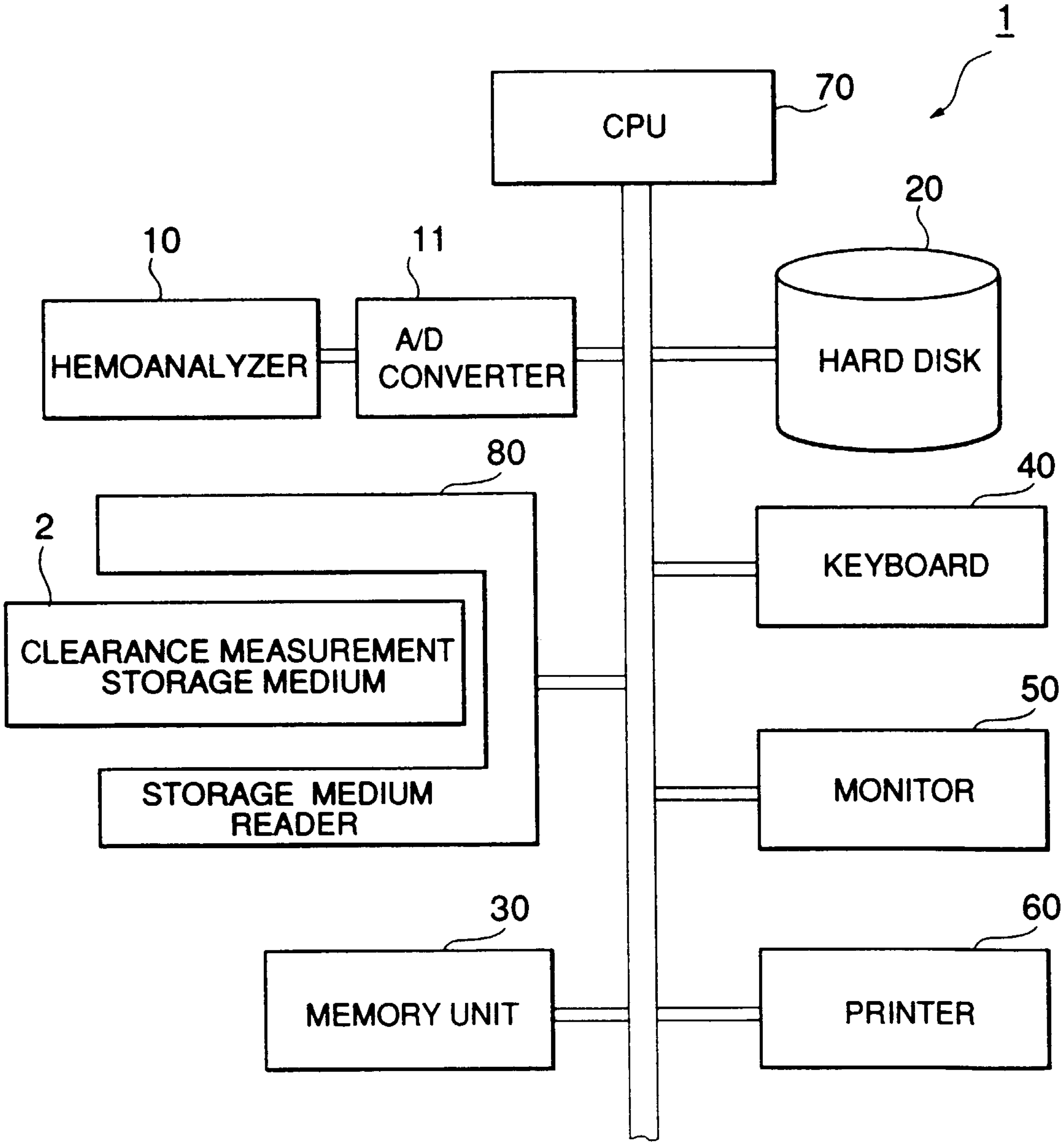
1
CPU
70
20
10
11
HEMOANALYZER
A/D CONVERTER
HARD DISK
80
40
2
KEYBOARD
CLEARANCE MEASUREMENT STORAGE MEDIUM
50
STORAGE MEDIUM READER
MONITOR
30
60
MEMORY UNIT
PRINTER

Fig.6

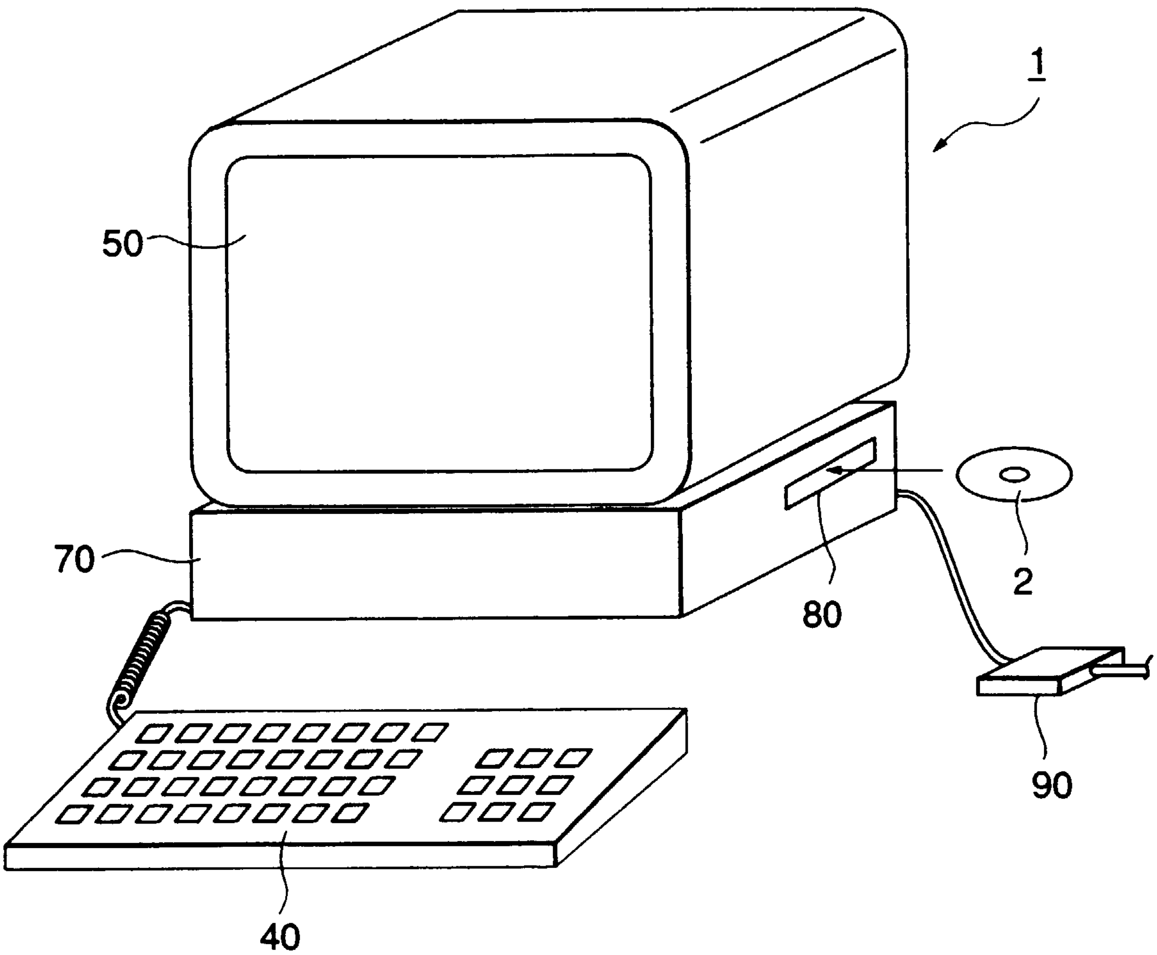

Fig.7

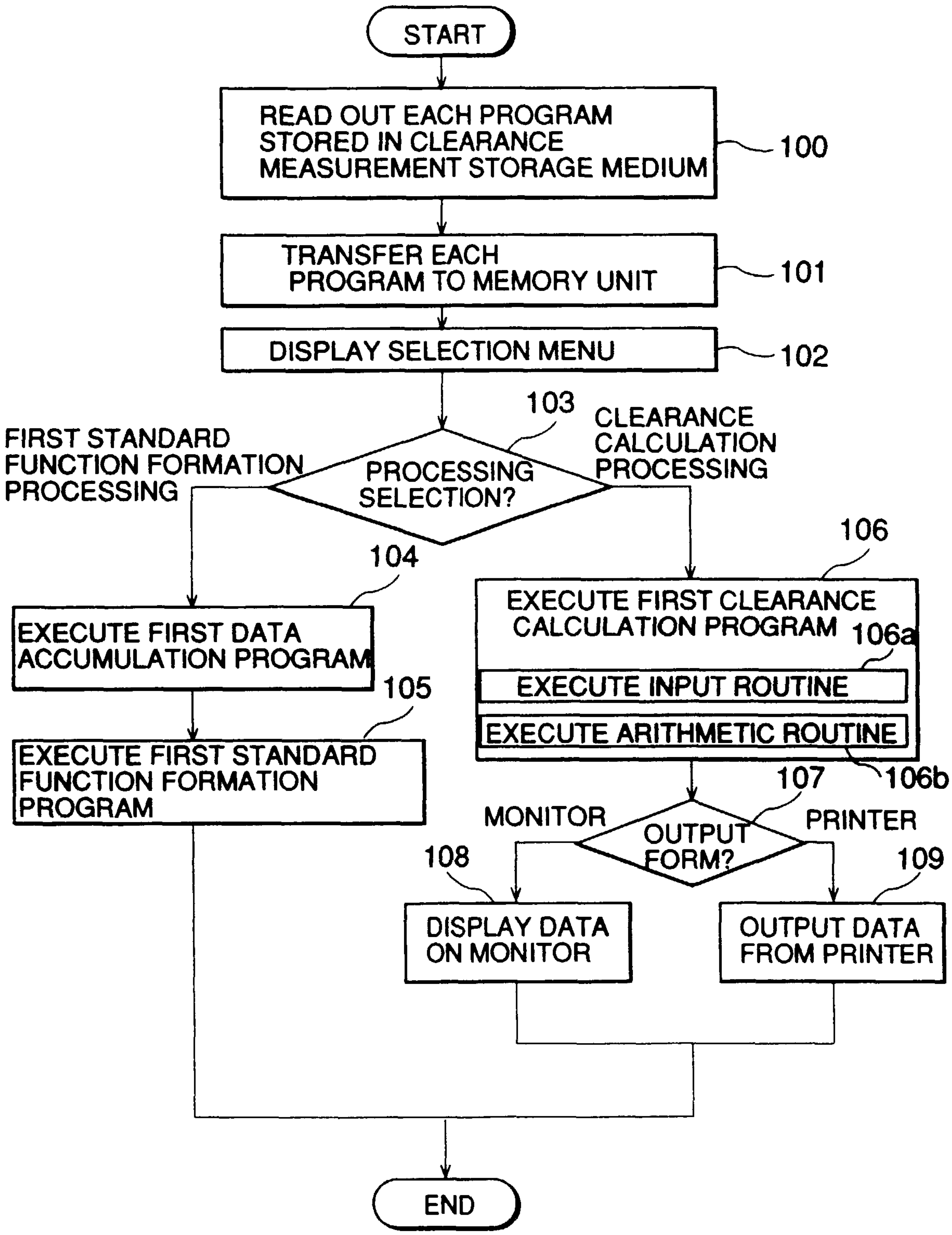
START
READ OUT EACH PROGRAM STORED IN CLEARANCE MEASUREMENT STORAGE MEDIUM
100
TRANSFER EACH PROGRAM TO MEMORY UNIT
101
DISPLAY SELECTION MENU
102
103
FIRST STANDARD FUNCTION FORMATION PROCESSING
PROCESSING SELECTION?
CLEARANCE CALCULATION PROCESSING
104
EXECUTE FIRST DATA ACCUMULATION PROGRAM
105
EXECUTE FIRST STANDARD FUNCTION FORMATION PROGRAM
106
EXECUTE FIRST CLEARANCE CALCULATION PROGRAM
106a
EXECUTE INPUT ROUTINE
EXECUTE ARITHMETIC ROUTINE
106b
107
MONITOR
OUTPUT FORM?
PRINTER
108
DISPLAY DATA ON MONITOR
109
OUTPUT DATA FROM PRINTER
END

Fig.8

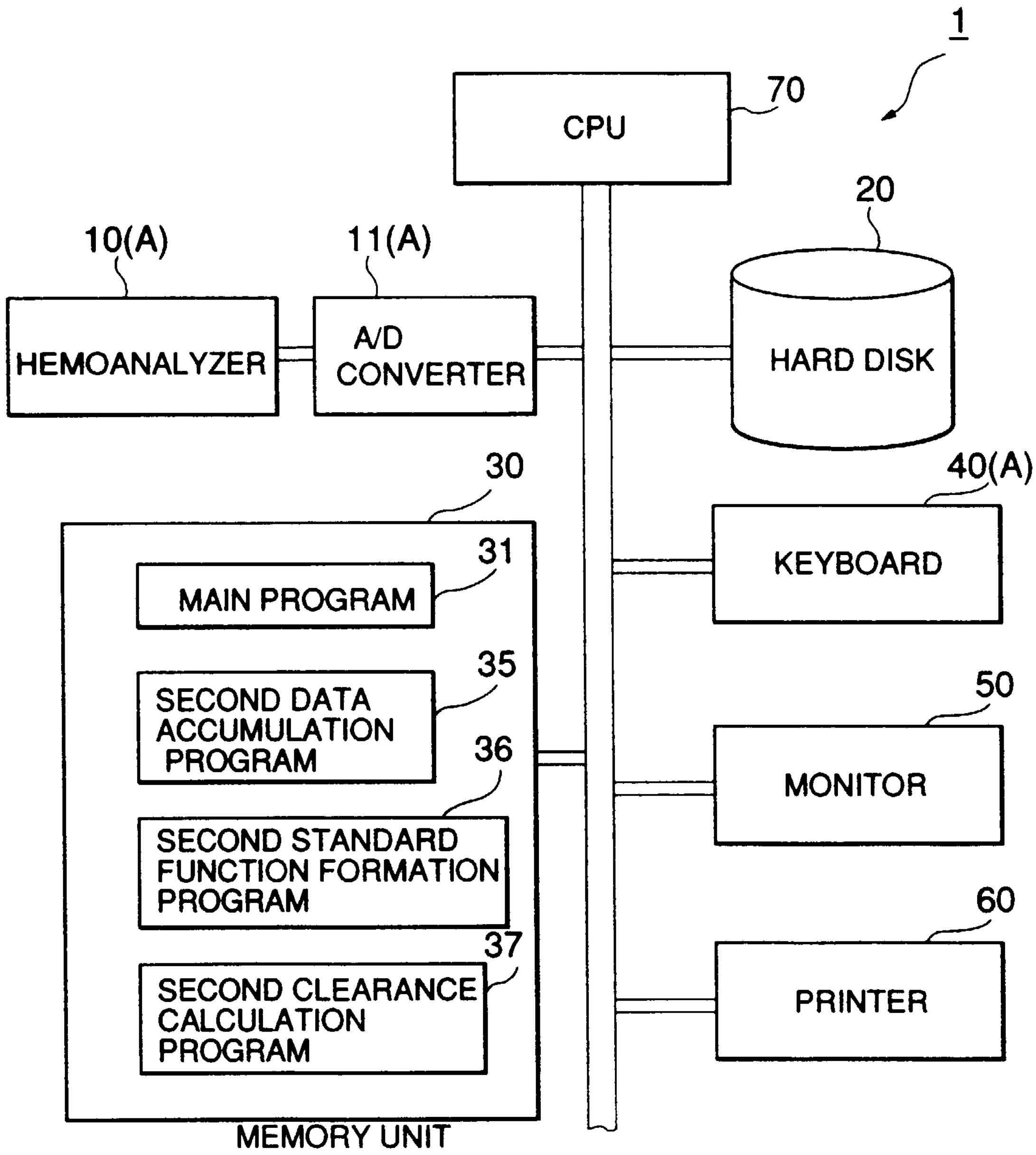
1
CPU
70
20
10(A)
11(A)
HEMOANALYZER
A/D CONVERTER
HARD DISK
30
40(A)
31
MAIN PROGRAM
KEYBOARD
35
SECOND DATA ACCUMULATION PROGRAM
50
36
MONITOR
SECOND STANDARD FUNCTION FORMATION PROGRAM
60
37
SECOND CLEARANCE CALCULATION PROGRAM
PRINTER
MEMORY UNIT

Fig.9

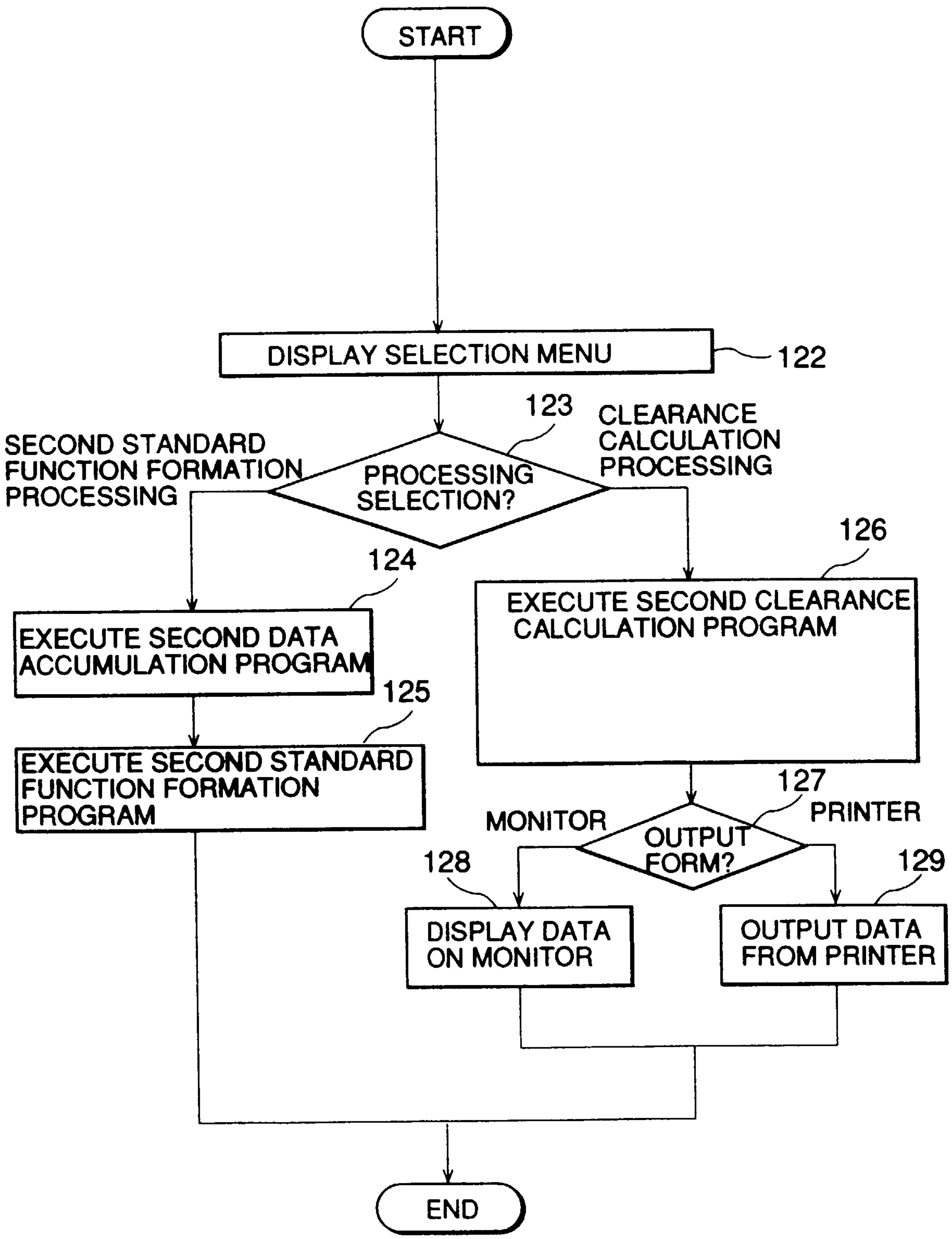
START
DISPLAY SELECTION MENU
122
123
PROCESSING SELECTION?
SECOND STANDARD FUNCTION FORMATION PROCESSING
CLEARANCE CALCULATION PROCESSING
124
EXECUTE SECOND DATA ACCUMULATION PROGRAM
125
EXECUTE SECOND STANDARD FUNCTION FORMATION PROGRAM
126
EXECUTE SECOND CLEARANCE CALCULATION PROGRAM
127
OUTPUT FORM?
MONITOR
PRINTER
128
DISPLAY DATA ON MONITOR
129
OUTPUT DATA FROM PRINTER
END

Fig.10

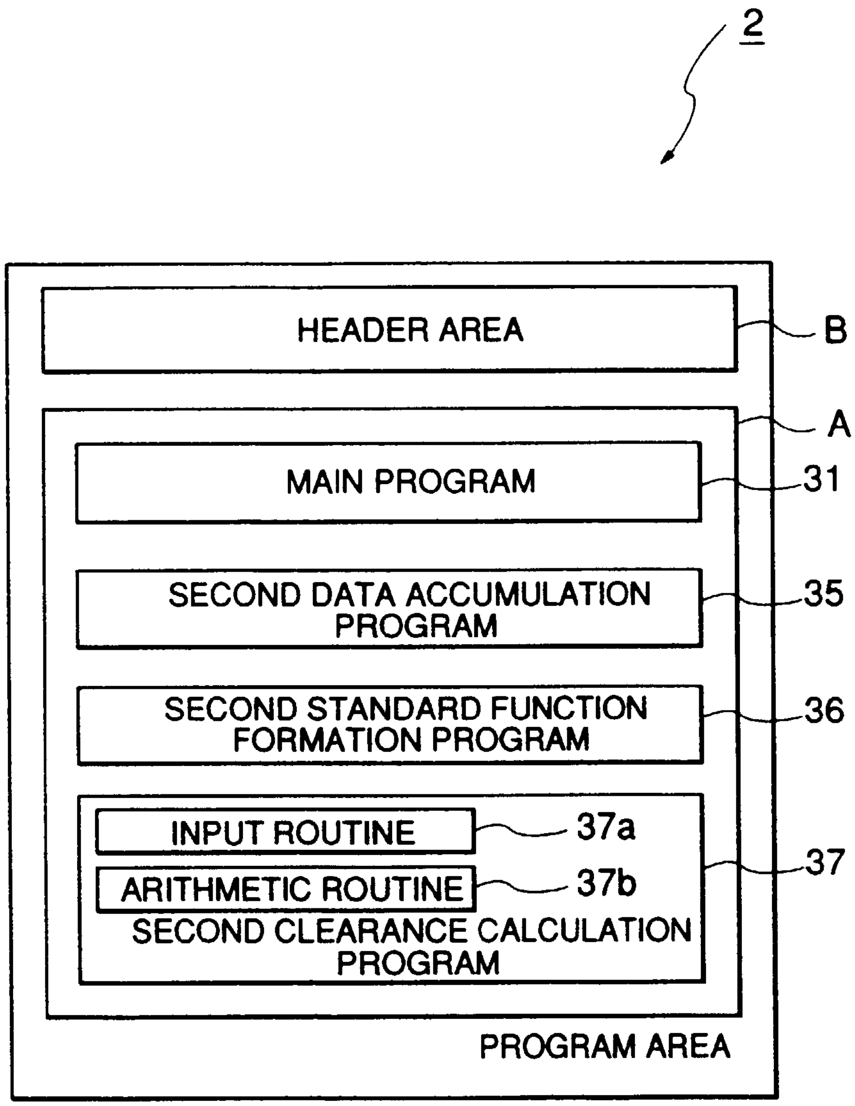


CLEARANCE MEASUREMENT STORAGE MEDIUM

Fig.11

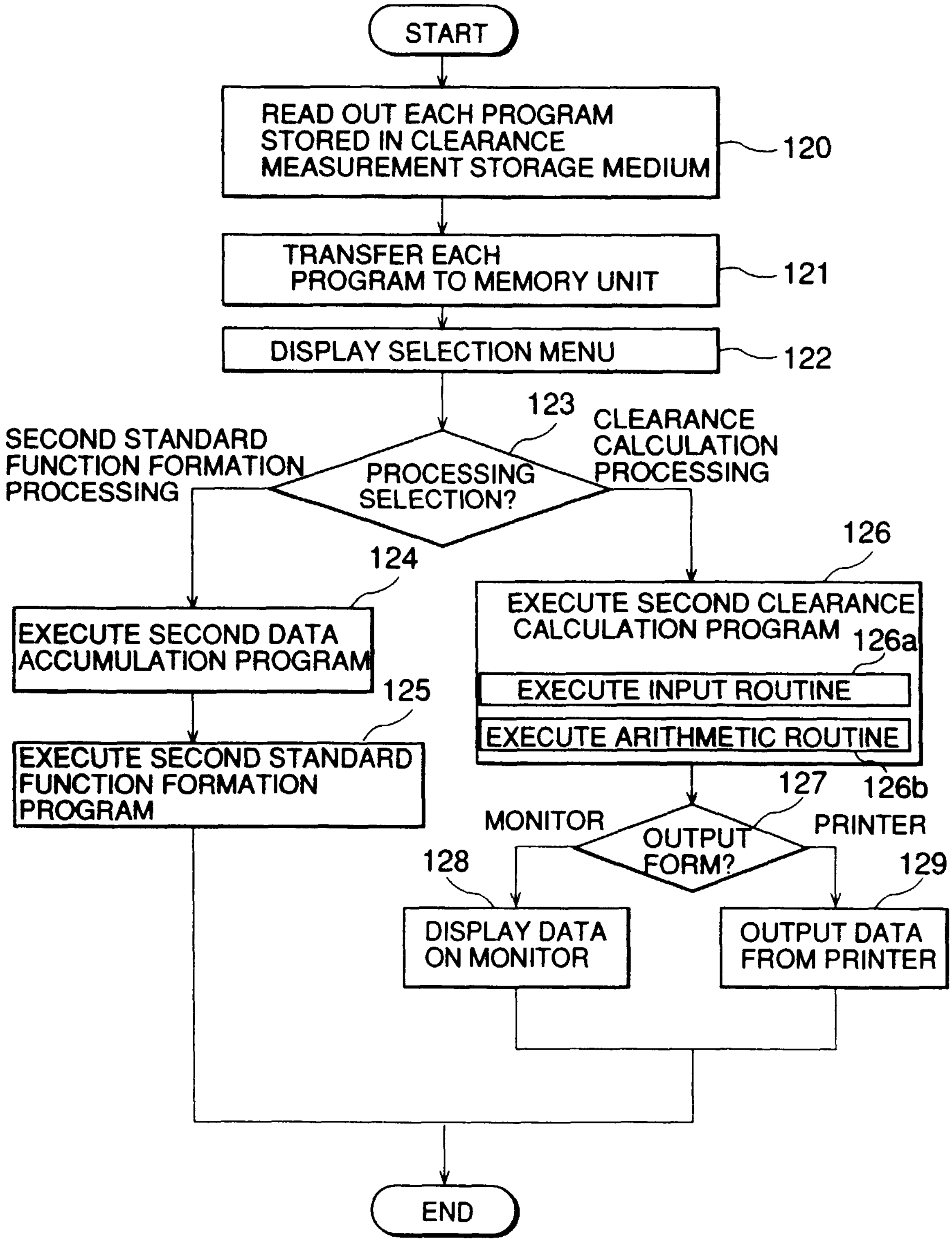
START
READ OUT EACH PROGRAM STORED IN CLEARANCE MEASUREMENT STORAGE MEDIUM
120
TRANSFER EACH PROGRAM TO MEMORY UNIT
121
DISPLAY SELECTION MENU
122
123
SECOND STANDARD FUNCTION FORMATION PROCESSING
PROCESSING SELECTION?
CLEARANCE CALCULATION PROCESSING
124
EXECUTE SECOND DATA ACCUMULATION PROGRAM
125
EXECUTE SECOND STANDARD FUNCTION FORMATION PROGRAM
126
EXECUTE SECOND CLEARANCE CALCULATION PROGRAM
126a
EXECUTE INPUT ROUTINE
EXECUTE ARITHMETIC ROUTINE
126b
127
MONITOR
OUTPUT FORM?
PRINTER
128
DISPLAY DATA ON MONITOR
129
OUTPUT DATA FROM PRINTER
END

Fig. 12

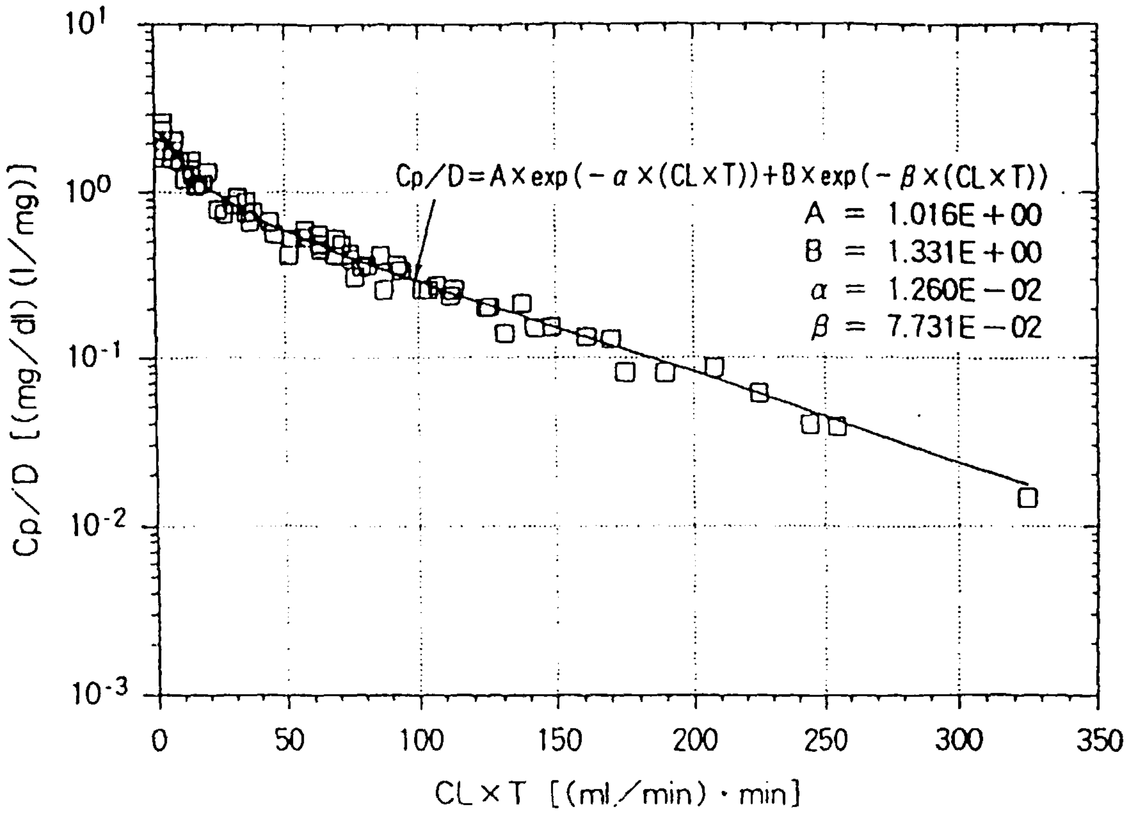
Cp/D=A×exp(−α×(CL×T))+B×exp(−β×(CL×T))
A = 1.016E+00
B = 1.331E+00
α = 1.260E−02
β = 7.731E−02
Cp/D [(mg/dl)(l/mg)]
CL×T [(ml/min)·min]
10¹
10⁰
10⁻¹
10⁻²
10⁻³
0
50
100
150
200
250
300
350

Fig. 13

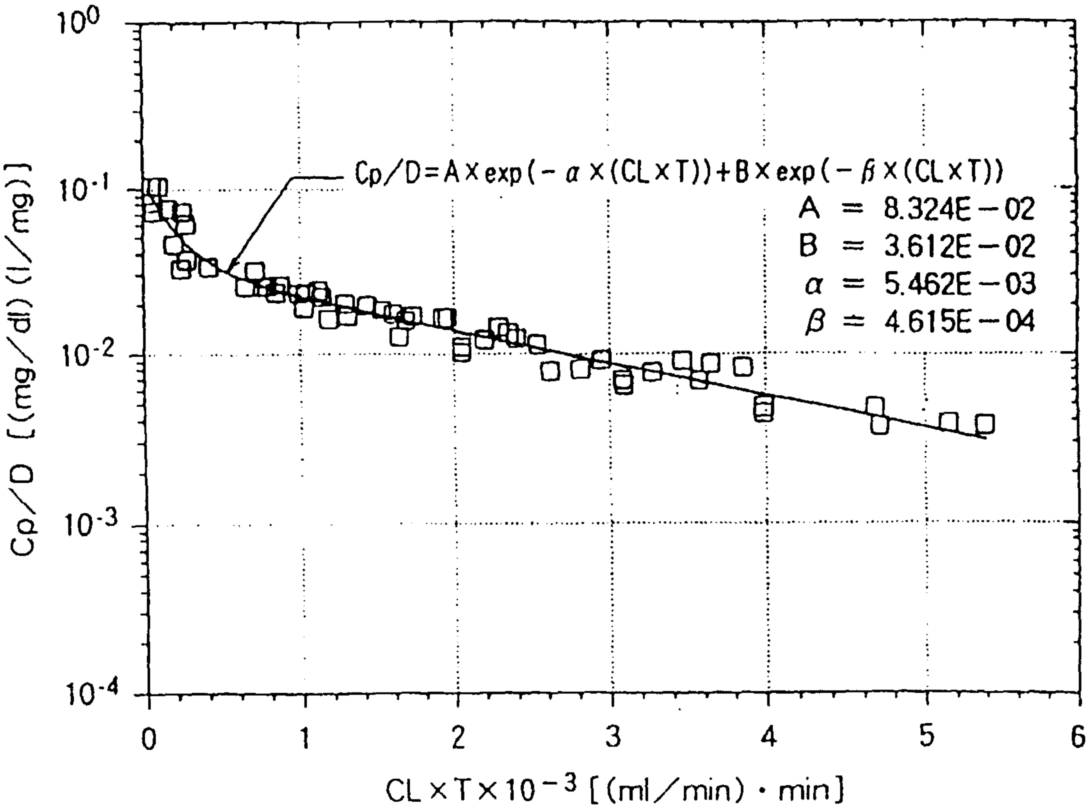

Cp/D=A×exp(−α×(CL×T))+B×exp(−β×(CL×T))
A = 8.324E−02
B = 3.612E−02
α = 5.462E−03
β = 4.615E−04
10⁰
10⁻¹
10⁻²
10⁻³
10⁻⁴
0
1
2
3
4
5
6
Cp/D [(mg/dl) (l/mg)]
CL×T×10⁻³ [(ml/min) · min]

Fig. 14

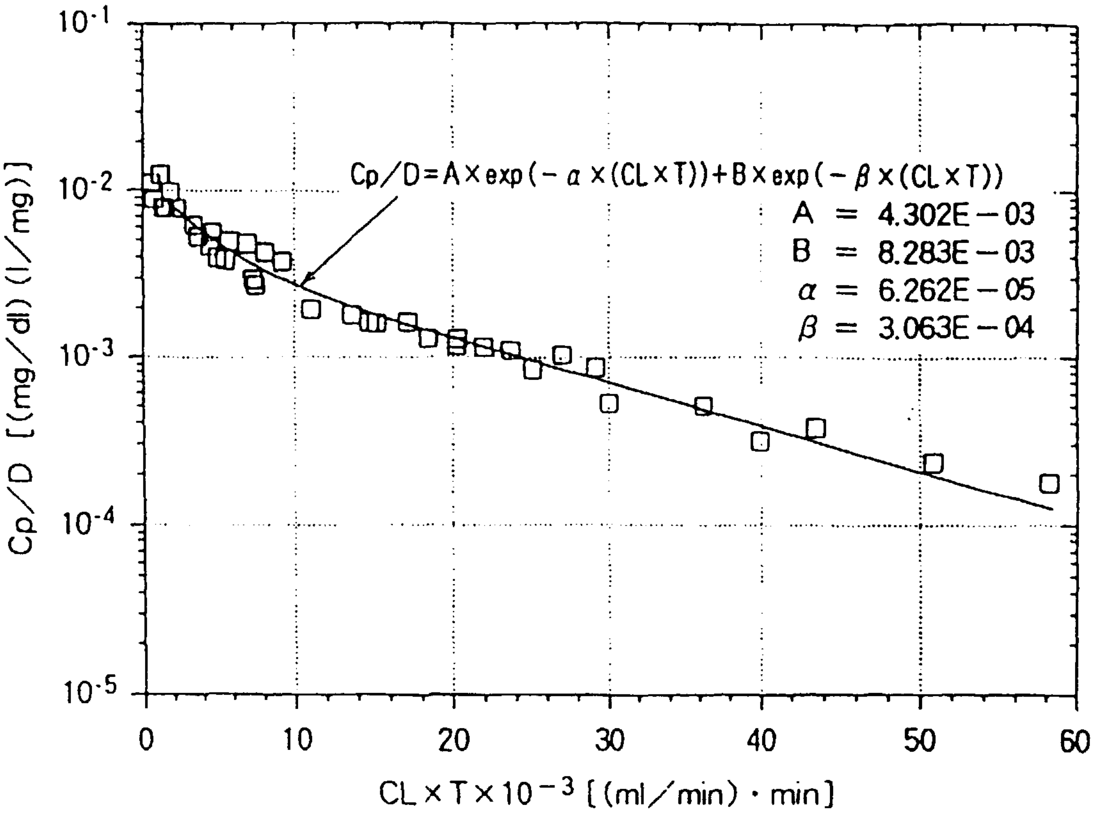
$10^{-1}$
$10^{-2}$
$10^{-3}$
$10^{-4}$
$10^{-5}$
Cp/D [(mg/dl) (l/mg)]
Cp/D=A×exp(−α×(CL×T))+B×exp(−β×(CL×T))
A = 4.302E−03
B = 8.283E−03
α = 6.262E−05
β = 3.063E−04
0
10
20
30
40
50
60
CL×T×10^−3 [(ml/min)・min]

Fig. 15

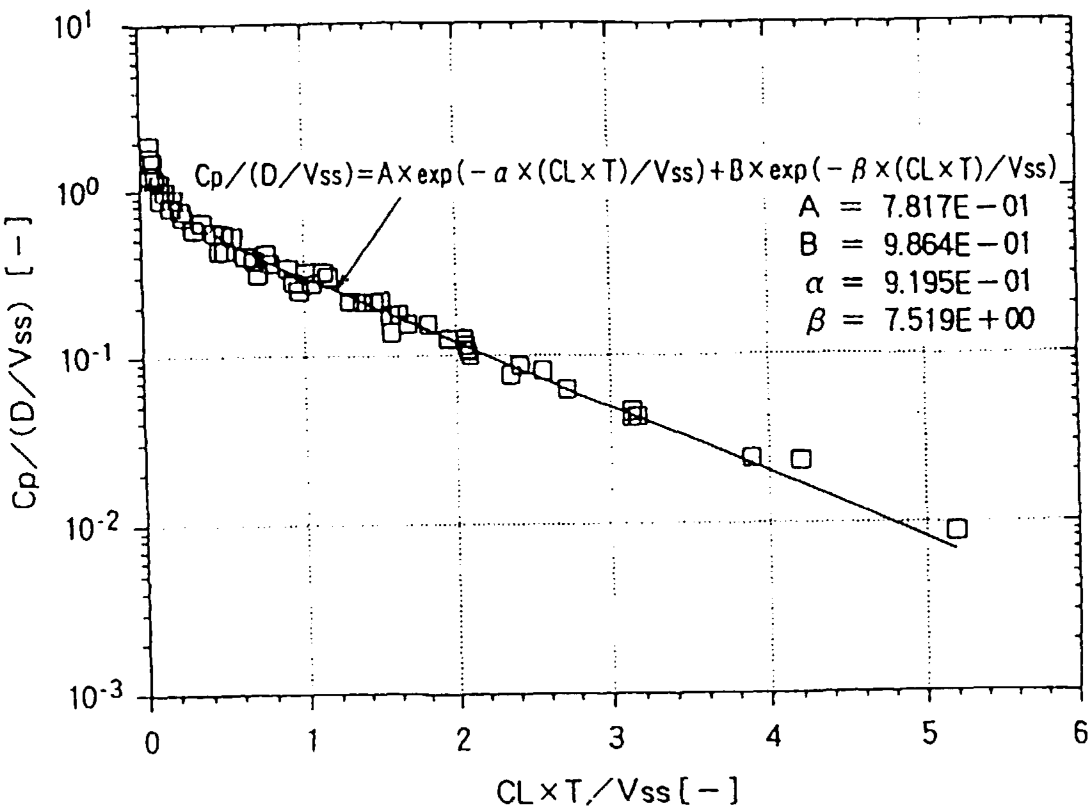
Cp/(D/Vss)=A×exp(−α×(CL×T)/Vss)+B×exp(−β×(CL×T)/Vss)
A = 7.817E−01
B = 9.864E−01
α = 9.195E−01
β = 7.519E+00
Cp/(D/Vss) [−]
CL×T/Vss [−]
10¹
10⁰
10⁻¹
10⁻²
10⁻³
0
1
2
3
4
5
6

Fig. 16

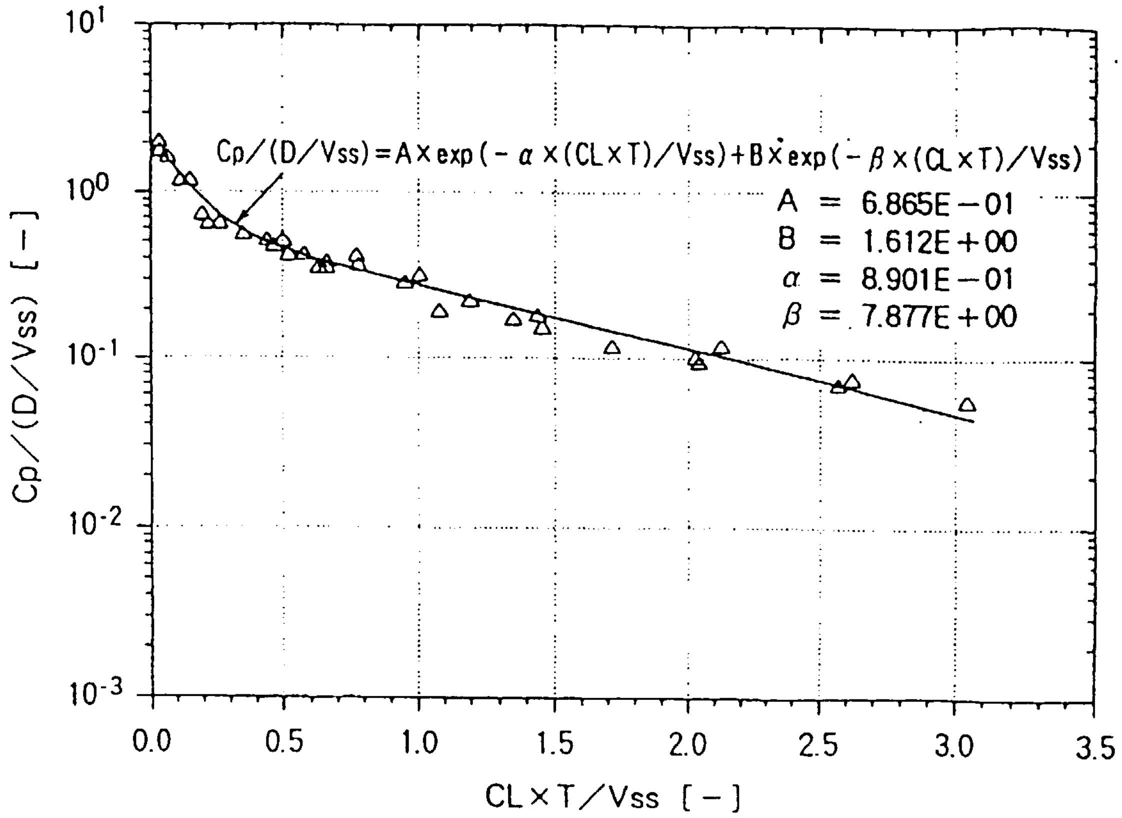
Cp/(D/Vss)=A×exp(−α×(CL×T)/Vss)+B×exp(−β×(CL×T)/Vss)
A = 6.865E−01
B = 1.612E+00
α = 8.901E−01
β = 7.877E+00
Cp/(D/Vss) [−]
CL×T/Vss [−]
10^1
10^0
10^-1
10^-2
10^-3
0.0
0.5
1.0
1.5
2.0
2.5
3.0
3.5

Fig. 17

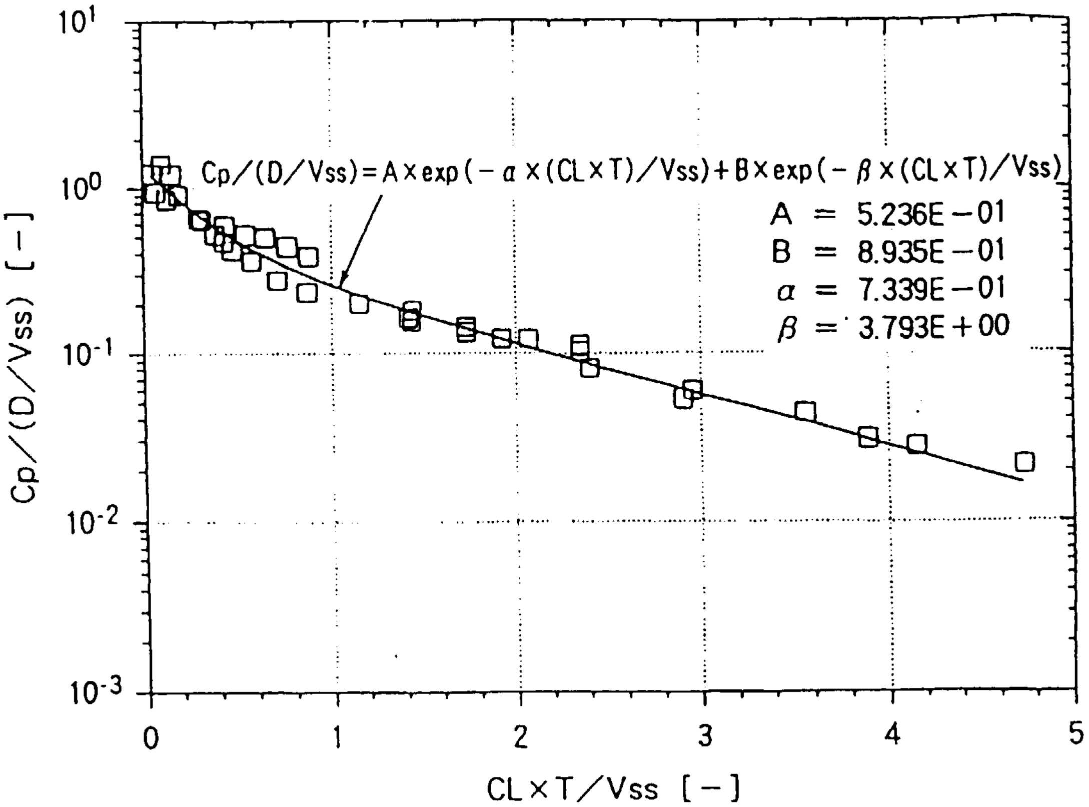
Cp/(D/Vss)=A×exp(−α×(CL×T)/Vss)+B×exp(−β×(CL×T)/Vss)
A = 5.236E−01
B = 8.935E−01
α = 7.339E−01
β = 3.793E+00
10^1
10^0
10^-1
10^-2
10^-3
Cp/(D/Vss) [−]
0
1
2
3
4
5
CL×T/Vss [−]

Fig. 18

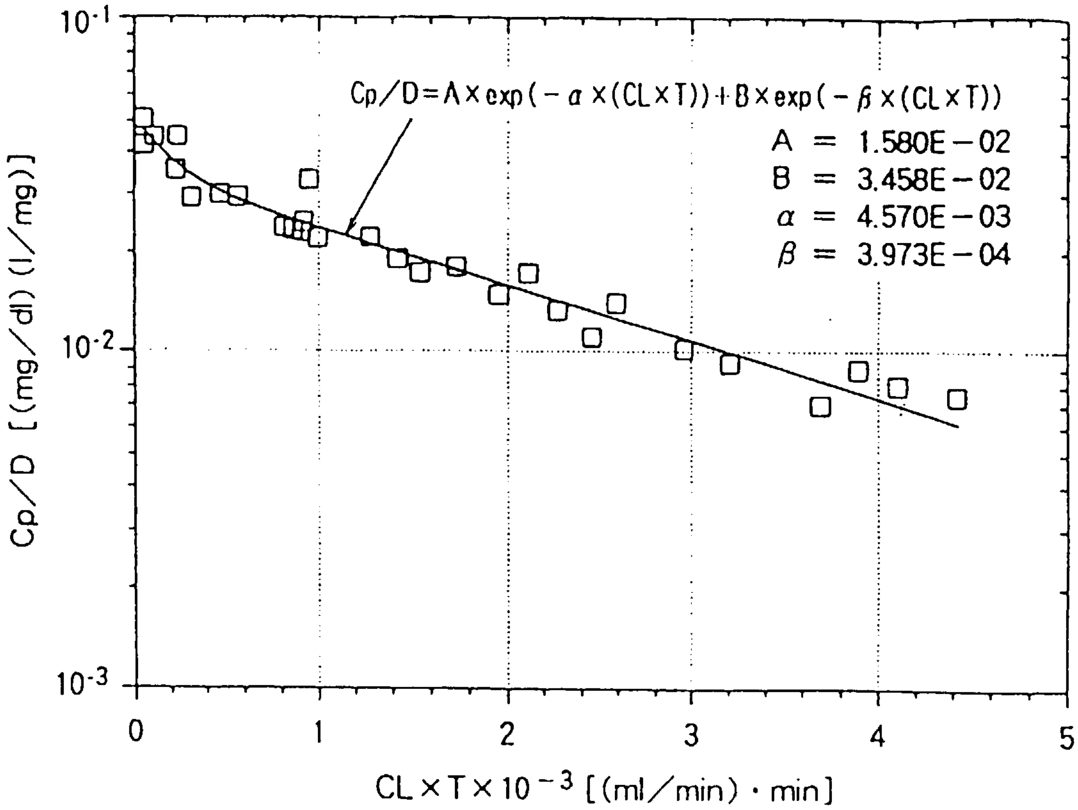
Cp/D=A×exp(−α×(CL×T))+B×exp(−β×(CL×T))
A = 1.580E−02
B = 3.458E−02
α = 4.570E−03
β = 3.973E−04
10^-1
10^-2
10^-3
Cp/D [(mg/dl) (l/mg)]
0
1
2
3
4
5
CL×T×10^−3 [(ml/min)・min]